# EUROPEAN PATENT SPECIFICATION

(11) **EP 0 947 585 B1**
(45) Date of publication and mention of the grant of the patent: **25.07.2001**
(21) Application number: 98105043.8
(22) Date of filing: 19.03.1998
(51) Int. Cl.: C12Q 1/56, G01N 33/86

(54) **Improved in vitro method, kits and reagents for screening for blood coagulation defects**
Verbesserte in vitro Verfahren, Testkits und Reagenzien zum Screening von Blutgerinnungsfehlern
Méthodes, trousses et réactifs améliorés pour le criblage de défauts de la coagulation sanguine

(43) Date of publication of application: 06.10.1999
(73) Proprietor: INSTRUMENTATION LABORATORY S.p.A., 20128 Milano (IT)
(72) Inventor: Rosén, Bert Steffen, 428 37 Kallered (SE); Hall, Christina Maria Yvonne, 439 32 Onsala (SE)
(74) Representative: TER MEER STEINMEISTER & PARTNER GbR

(56) References cited:
- WO-A-93/10262
- US-A- 5 001 069
- M.M.BERNARDO, D.E.DAY, S.T.OLSON, J.D.SHORE: "Surface-independent Acceleration of Factor XII Activation by Zinc Ions" JOURNAL OF BIOLOGICAL CHEMISTRY, vol. 268, no. 17, 15 June 1993, pages 12468-12476, XP002067665
- F.SEKIYA, T.YAMASHITA, H.ATODA, Y.KOMIYAMA, T.MORITA: "Regulation of the Tertiary Structure and Function of Coagulation Factor IX by Magnesium(II) Ions" JOURNAL OF BIOLOGICAL CHEMISTRY, vol. 270, no. 24, 16 June 1995, pages 14325-14331, XP002067666
- M.J.HEEB, A.GRUBER, J.H.GRIFFIN: "Identification of Divalent Metal Ion-dependent Inhibition of Activated Protein C by alpha2-Macroglobulin and alpha2-Antiplasmin in Blood and Comparisons to Inhibition of Factor Xa, Thrombin, and Plasmin" JOURNAL OF BIOLOGICAL CHEMISTRY, vol. 266, no. 26, 15 September 1991, pages 17606-17612, XP002067667
- H.A.LIEBMAN, B.C.FURIE, B.FURIE: "The Factor IX Phospholipid-binding Site Is Required for Calcium-dependent Activation of Factor IX by Factor X" JOURNAL OF BIOLOGICAL CHEMISTRY, vol. 262, no. 16, 5 June 1987, pages 7605-7612, XP002067668
- J.D.SHORE, D.E.DAY, P.E.BOCK, S.T.OLSON: "Acceleration of Surface-Dependent Autocatalytic Activation of Blood Coagulation Factor XII by Divalent Metal Ions" BIOCHEMISTRY, vol. 26, no. 8, 1987, pages 2250-2258, XP002067669
- S.BUTENAS, J.H.LAWSON, M.KALAFATIS, K.G.MANN: "Cooperative Interaction of Divalent Metal Ions, Substrate, and Tissue Factor with Factor VIIa" BIOCHEMISTRY, vol. 33, no. 11, 1994, pages 3449-3456, XP002067670

## Description

### Field of the Invention

The present invention relates to in vitro photometric methods, kits and reagents for qualitative screening and quantitative determination of the functional activity of components of the Protein C anticoagulant pathway of blood coagulation, comprising measuring the conversion rate of an exogenous substrate by an enzyme, the activity of which is related to the Protein C anticoagulant activity, in a blood sample of a human comprising coagulation factors and said exogenous substrate after at least partial activation of coagulation through the intrinsic, extrinsic or common pathway and triggering coagulation by adding calcium ions; and comparing said conversion rate with the conversion rate of a normal human blood sample determined in the same way,

### Background of the Invention

Maintenance of a proper hemostasis is the result of a careful balance between pro- and anticoagulant activities. After a trauma, coagulation is triggered primarily through activation of coagulation Factors IX and X (FIX, FX) by tissue factor (also denoted tissue thromboplastin) and Factor VII (FVII) followed by generation of thrombin, which in turn cleaves fibrinogen to form soluble fibrin. After crosslinking by Factor XIII, a three-dimensional insoluble gel clot is obtained which prevents further blood losses.

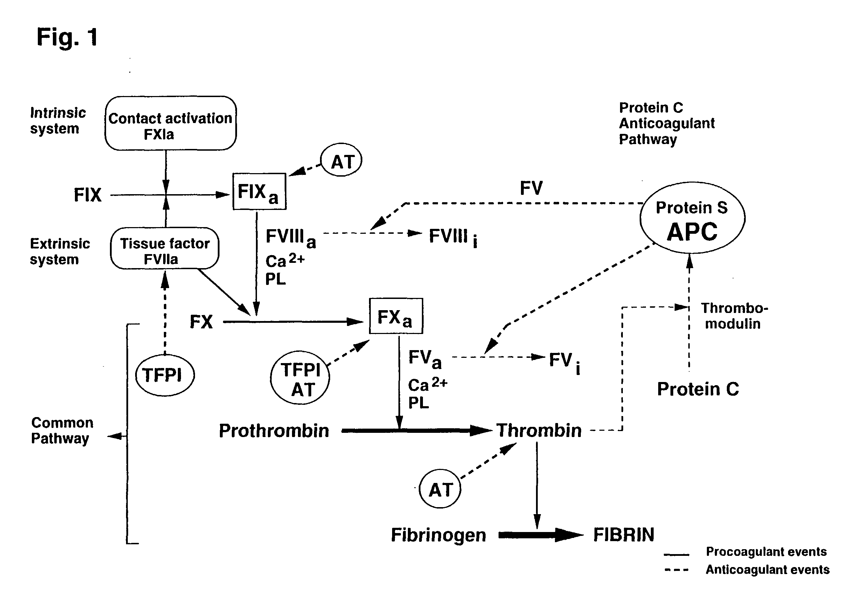

Regulation of this highly potent system - shown schematically in Figure 1 of the drawings - is accomplished by a balanced relation between procoagulant events (shown with solid line arrows) and anticoagulant events (shown with dashed line arrows). The anticoagulant events comprise 1) inhibition of already formed thrombin by antithrombin (AT) and α₂-macroglobulin and 2) prevention of further thrombin formation by the Protein C anticoagulant pathway. Here, activated Protein C (APC) inactivates the coagulation proteins Factor VIII and Factor V in their activated forms (FVIIIa, FVa) through proteolytic cleavage. In addition inhibition of generated Factor Xa is also accomplished by antithrombin and tissue factor pathway inhibitor (TFPI), the latter also inhibiting the tissue factor/Factor VIIa complex. Factor Villa and Factor Va act as cofactors in the activation of Factor X and prothrombin, respectively, and increase the reaction rates of these processes about 1000-fold each. Thus, these cofactors act as potent stimulators of the coagulation. Their inactivation by APC therefore essentially stops further thrombin generation, thus providing a strong anticoagulant effect. Protein S and also Factor V act as cofactors to activated Protein C (APC).

As shown in Figure 1, the activation of coagulation through the intrinsic or extrinsic systems results in the activation of Factor X, a key component in the final common pathway. In the intrinsic system, the initial event is the activation of contact factors (Factor XII, prekallikrein) followed by the activation of Factor XI, which in turn activates Factor IX. In the extrinsic system, Factor IX and Factor X are activated by the tissue factor/Factor VIIa complex.

As Figure 1 as well shows, calcium ions have to be present in several of these reactions. The activation of Factor X by Factor IXa and of prothrombin by Factor Xa requires procoagulant phospholipids. In vivo, this is provided by the membrane surface of activated platelets; in vitro by platelet extracts, purified phospolipids or crude phospholipid extracts from suitable sources. The total and free calcium ion concentration in native plasma is about 2.4 and and 1.2 mmol/L, respectively. Typically, calcium ion concentrations used in analytical methods for determination of coagulation or anticoagulation factors are in the range 1.5 -10 mmol/L. The concentrations of other metal ions in plasma are lower, typical values for total concentration being 1 mmol/L for Mg²⁺ and 5 - 40 µmol/L for Zn²⁺, Cu²⁺ and Mn²⁺.

Defects in the Protein C anticoagulant pathway may cause a risk of thrombosis due to a decreased capacity to prevent thrombin formation. Such defects may be due to deficiencies in the activity of Protein C and/or its cofactor Protein S. Another recently detected defect is a point mutation in the Factor V gene (G→A) at nucleotide 1691, resulting in the amino acid substitution Arg (R) → Gln (Q) at position 506 in FactorV/FactorVa, denoted FV:Q⁵⁰⁶ or Factor V Leiden. Heterozygosity and homozygosity for this mutation are often denoted FV:R506Q and FV:Q506Q, respectively. This mutation is at one of the three APC cleavage sites (amino acids 306, 506, 679) in Factor Va, which confers an impairment of its degradation by activated Protein C (APC), denoted APC resistance.

APC resistance is to be considered a blood coagulation disorder recognized by an abnormally low anticoagulant response to activated Protein C (APC) and the determination of said APC resistance may be used to screen for and diagnose thromboembolic diseases, such as hereditary thrombophilia or for determining the risk for a human to acquire a manifestation of this blood coagulation disorder (Dahlbäck B., *EP-608 235*).

Hence, there is a need to investigate these components of the Protein C anticoagulant pathway in the evaluation of thrombotic patients and potentially also to screen for abnormalities of Protein C, Protein S and Factor V anticoagulant activity in situations connected with an increased risk of thrombosis such as before surgery, during trauma and during pregnancy or in connection with the use of oral contraceptive pills or hormone replacement therapy. Currently, clotting and/or chromogenic assays are available for analysis of Protein C and Protein S activity as well as for the detection of APC resistance, which to at least 90% is due to the Factor V mutation at position 506.

Protein C activity is typically measured after activation of the endogenous Protein C, contained in a plasma sample, by thrombin or by a snake venom enzyme from Agkistrodon contortrix contortrix (Stocker K., *EP 203 509),* commercially available as the reagent Protac® C (Pentapharm AG, Basel, Switzerland). The concentration of Protac® C in the activation mixture is typically about 0.1 U/mL or above since otherwise an insufficient activation of Protein C may be obtained (Martinoli J., Stocker *K.Thromb Res* 43, 253-264 (1986); Mc Call F. et al.Thromb Res 45, 681-685 (1987)).

After activation by Protac® C, the protein C activity is determined with a clotting or chromogenic assay (Bertina R. *Res Clin Lab,* 20, 127-138 (1990); Marlar R., Adcock DM. *Hum Pathol* 20, 1040-1047 (1989), Rosén S., EP 486 515). In clotting methods, coagulation is triggered through the intrinsic pathway by using APTT reagents or through the extrinsic pathway with the use of tissue factor. In both cases calcium ions are added to a final concentration of usually 5-10 mmol/L. Commercial kits and reagents are available for determination of Protein C activity such as Acticlot™ C (American Diagnostica), Stachrom Protein C (Diagnostica Stago), Staclot Protein C (Diagnostica Stago), Coamatic® Protein C (Chromogenix AB) and Protein C Activator (Behring Diagnostica).

The activation of Protein C by thrombin is stimulated about 1000-fold by thrombomodulin, an endothelial cell membrane protein (Esmon CT and Owen WG. *Proc Natl Acad Sci* 78, 2249-2252 (1981)). The use of thrombin/thrombomodulin as activator of Protein C for analysis of Protein C and/or Protein S activity in plasma samples, utilizing a photometric method, is also known (Pittet J-L. and Aiach M., *FR Pat Appl. 2689 640-A1*).

Protein S activity is determined from its stimulation of the activity of APC in its degradation of Factor Va and/or Factor VIlla. Typically, in such assays a standardized amount of APC is added to a plasma sample or activation of endogenous protein C is performed whereafter the clotting time is determined after a simultaneous or separate coagulation activation via the intrinsic system using an APTT reagent, via the extrinsic system using tissue factor or by Factor Xa (Bertina R., loc cit; Preda L. et al,Thromb Res 60, 19-32 (1990); D'Angelo S. et al, Thromb Res 77, 375-378(1995)). Chromogenic activity assays for protein S have also been published, utilizing Factor IXa as activator and monitoring Factor Xa generation (van de Waart P, Woodhams BJ, *EP 567 636*) or thrombin generation (Rosen S., *EP 486 515* ). In all these methods calcium ions are added as mentioned above.

The Factor V mutation (FV:Q⁵⁰⁶) in the Factor V molecule may be detected with molecular biology methods based upon the use of the polymerase chain reaction (PCR) technique (Bertina RM et al, *Nature* 369, 64-67 (1994)) or by methods in which the functional activity of APC is determined. Such activity methods may be coagulation-based (Dahlbäck B, *EP 608 235* ; Rosén et al, *Thromb Haemost* 72, 255-260 (1994)) and include the use of predilution of sample plasma with a plasma with no or a low Factor V activity (Dahlbäck B, *EP-A-94 905 908.3 ;* Jorquera JI et al, *Lancet* 344, 1162 - 1163 (1994); Svensson PJ et al, *Thromb Haemost* 77, 332-335 (1997)). The latter assay principle, i.e. a coagulation-based assay using predilution of sample plasma is also utilized in a commercial product, Coatest® APC Resistance V (Chromogenix AB). Alternatively, chromogenic methods may be used (Dahlbäck B, *EP 608 235*; Rosén et al, *Thromb Haemost* 73, 1364, Abstract 1778 (1995), Nicolaes GAF et al, *Thromb Haemost* 76, 404-410 (1996)).

Since genetic defects in the Protein C anticoagulant pathway are found in about 25% of unselected patients with venous thromboembolism (VTE) and in about 50% of patients with thrombophilia, i.e. patients from families with an increased tendency to VTE, there is a need for a single test which detects all such abnormalities with a high sensitivity and specificity, i.e. a global (overall) test. One concept for a global test is based upon the activation of Protein C in plasma with Protac® C and activation of coagulation via the intrinsic or extrinsic pathway (Bartl et al, *USP 5, 001, 069* ; Kraus M, *EP-A- 696 642*). Results obtained with a commercial kit application of this test, ProC Global (Behring Diagnostica, Marburg, Germany), in which intrinsic activation of coagulation is accomplished through addition of an APTT reagent, show a sensitivity for Protein C deficiency, Protein S deficiency and for FV:Q506 of, respectively, about 90%, 50-80% and more than 90% on analysis of healthy individuals and thrombotic patients (Dati F et al, *Clin Chem* 43, 1719-1723 (1997); Ruzicka K et al, *Thromb Res* 87, 501-510 (1997)). The specificity is about 50% in thrombotic cohorts (Dati F et al, loc cit), i.e. a substantial proportion of positive results are obtained, which can not be linked to known defects in components in the Protein C anticoagulant pathway, such as in protein C, protein S and Factor V. Thus, this test lacks sufficient specificity.

Furthermore, results from analysis of pregnant women lacking any of the known defects in the Protein C anticoagulant pathway are clearly different from analysis of normal healthy individuals (Rangård B, Wagner C, *Annals Hematol* 74: Supplement II, Abstract 74, A77 (1997); Siegemund A et al, *Annals Hematol* 74: Supplement II, Abstract 188, A105 (1997)), which necessitates separate ranges for these cohorts. This as yet uncharacterized interference limits the general applicability of the test. An alternative global method for the detection of defects in the Protein C anticoagulant pathway, based upon activation of endogenous plasma Protein C by Protac C utilizes tissue factor as trigger of the coagulation (Preda L et al, *Blood Coag Fibrinol* 7, 465-469 (1996)). Also here, the sensitivity of the method is limited, especially for Protein S. Furthermore, different sample categories, e.g. pregnant and non-pregnant women, may require different approaches for evaluation of the results due to interferences not only related to components in the Protein C anticoagulant pathway.

For a global test to be useful as a screening test for known inherited defects in the Protein C anticoagulant pathway, i.e. Protein C deficiency, Protein S deficiency and the FV:Q506 mutation, the sensitivity should be high, at least 90%, for all these defects. Furthermore the specificity should be high, above 60%, suitably above 70% and preferably above 80% in order to considerably reduce the number of false positive results. The state-of-the-art methods do not provide a satisfactory solution to these requirements. For the development of improved methods for the specific determination of Protein C and Protein S activity and for determination of mutations in Factor V which affects its anticoagulant activity, it is also desirable to improve the resolution and specificity of these methods.

There is also a need to improve the stability of different components used as separate reagents or as reagents in kits comprising such methods.

Thus, the technical problem underlying the present invention is the provision of in vitro methods with improved sensitivity and specificity for screening and for the specific detection of defects in the Protein C anticoagulant pathway of blood coagulation in a human. A further recognized problem is to improve the stability of components in such methods.

### Brief Description of the Invention

It has now been unexpectedly found that the above technical problems can be solved based upon the surprising finding that the addition of low levels of ions of divalent metals, such as Mg²⁺, Mn²⁺, Zn²⁺, Ni²⁺, Sr²⁺, Cu²⁺ ions or of the monovalent Cu⁺ ion, in the presence of calcium ions enhances the anticoagulant activity of the Protein C anticoagulant pathway and provides for a high resolution between different levels of Protein C activity and Protein S activity, respectively, and a high discrimination for the presence of the FV:Q⁵⁰⁶ mutation, resulting in an improved sensitivity and specificity for detection of defects in components of the Protein C anticoagulant pathway with photometric and/or clotting methods. Thus, the invention also constitutes a new excellent global method for the Protein C anticoagulant pathway. In addition to that, divalent metal ions provide for an unexpected improvement of the stability of the reagents used either when used separately or when used in test kits for determining the anticoagulant activity of components of the Protein C anticoagulant pathway in blood samples with photometric and/or clotting methods.

Thus, the above problems are solved by the characterizing features of the attached claims.

Subject-matter of the present invention thus is an in vitro photometric method for qualitative screening and quantitative determination of the functional activity of components of the Protein C anticoagulant pathway of blood coagulation, comprising measuring the conversion rate of an exogenous substrate by an enzyme, the activity of which is related to the Protein C anticoagulant activity, in a blood sample of a human comprising coagulation factors and said exogenous substrate after at least partial activation of coagulation through the intrinsic, extrinsic or common pathway and triggering coagulation by adding calcium ions; and comparing said conversion rate with the conversion rate of a normal human blood sample determined in the same way, said method being characterized by adding further metal(s) ions to said sample.

The present invention is thus concerned with a novel in vitro method for screening for, in a human, defects in the Protein C anticoagulant pathway due to e.g. Protein C deficiency, Protein S deficiency and Factor V mutations such as the FV:Q506 mutation, or other Factor V defects related to APC resistance and/or APC cofactor activity. Such a method may be designed for the specific detection of either of Protein C deficiency, Protein S deficiency or of mutations in Factor V/Factor Va which affect the cleavage rate by APC. One preferred embodiment of the present invention comprises a global test for the Protein C anticoagulant pathway.

The term "metal(s) ions" stands for the fact that ions of one or more of said further metals may be present. Preferred ions of said further metals are divalent metal ions or the ions of monovalent copper, such as Mg²⁺, Mn²⁺, Zn²⁺, Cu²⁺, Ni²⁺, Sr²⁺, and/or Cu⁺ ions.

The term "blood sample" is defined to cover a blood sample, such as whole blood, or a blood derived sample such as a blood plasma sample or a blood serum sample.

The term "photometric assay" is defined to cover colorimetric, fluorimetric and luminometric assay methods.

The term "coagulation factors" stands for such factors comprising components in the intrinsic, extrinsic and common pathway (procoagulant events see Figure 1) and in the Protein C anticoagulant pathway (anticoagulant events see Figure 1) and being either solely endogenous, i.e. being inherent in the blood sample, or comprising also the addition of such exogenous factors. Furthermore, phospholipid(s) may be added in the method when utilizing any of the intrinsic, extrinsic or common pathway for activation of coagulation.

Said novel method will thus allow improved screening and diagnosing of defects in the Protein C anticoagulant pathway in investigation of patients with thromboembolic diseases such as deep venous thrombosis and/or pulmonary embolism. In case a patient belongs to a family with hereditary thrombophilia, said novel method is also suitable for investigation of family members of such a patient in order to determine the possible inheritance of defects within said pathway. Said novel method is also suitable for diagnosing of defects in the Protein C anticoagulant pathway in investigation of patients before surgery, patients with trauma or in pregnant women or in women receiving oral contraceptive pills or hormone replacement therapy such as oestrogen therapy. Furthermore, global methods may be designed to allow the detection of known defects and of hitherto unrecognized defects in the Protein C anticoagulant pathway. The invention may also allow the design of specific photometric and/or clotting methods for such as yet unrecognized defects in said pathway.

Any of the above methods encompasses monitoring of conversion of a photometric exogenous substrate for either Factor Xa or thrombin, containing as leaving group a chromophore, a fluorophore or a luminophore. Examples of such photometrically measurable leaving groups are p-nitroaniline (pNA, chromophore) for use in colorimetric methods, e.g. naphthylamine and coumarin derivatives such as methylcoumarine, aminoisophthalic acid and its derivatives (fluorophores) for use in fluorimetric methods and e.g. isoluminolamide (luminophore) for use in luminometric methods.

The invention is most unexpected in light of the present knowledge in the field which, in fact, teaches that several divalent ions *increase the procoagulant activity* of certain vitamin K-dependent coagulation factors in the presence of calcium ions (see procoagulant events in Figure 1), so that it could not be expected that the addition of such further metal(s) ions could enhance tests relating to the *anticoagulant activity,* specifically in the Protein C anticoagulant pathway.

Thus, it is known that Mg²⁺ stimulates the activity of Factor IXa (Byrne et al, *JBiol Chem* 1980; 255: 1430-1435; Sekiya F et al, *J Biol Chem* 1995; 270: 14325-14331; Sekiya F et al, *J Biol Chem* 1996; 271: 8541-8544; Morita T et al. *Thromb Haemost* 1997; 78, Supplement: 430, Abstract PS-1755) and also enhances the activation rate of Factor IX by FXIa and tissue factor (Sekiya Fetal, *loc sit 1995*, *loc cit* 1996; Morita T et al., *loc cit*). It was also shown that neither Protein C nor prothrombin, Factor VII and Factor X are responsive to Mg²⁺ (Sekiya F et al, *loc cit 1995* ). Mg²⁺ has been shown to also stimulate the prothrombin activation by Factor Xa, phospholipid and calcium ions (Prendergast FG and Mann KG, *J Biol Chem* 1977; 252: 840-850), an effect which, however, may not be pronounced at calcium ion concentrations above 1 mmol/L (Sekiya F et al, *loc cit 1995*). Furthermore, it has been shown that Factor IX has a unique binding site for Mn²⁺ (Amphlett GW et al, *J Biol Chem* 1978; 253: 6774-6779), which site has been suggested to be identical with the Mg²⁺ binding site (Sekiya F et al, *loc cit 1995*).

Mn²⁺ ions have also been shown to enhance the binding of Factor IX to procoagulant phospholipids in the presence of calcium or Sr²⁺ ions, the latter thus also having a procoagulant effect (Liebman et al, *J Biol Chem* 1987; 262, 7605-7612).

Furthermore, Mg²⁺ and Mn²⁺ ions have been shown to increase the amidolytic activity of Factor VIIa, i.e. the cleavage rate of low molecular weight synthetic peptide substrates (Butenas S et al, *Biochemistry* 1994; 33: 3449-3456; Persson E, Petersen LC, *Eur J Biochem* 1995; 234: 293-300) whereas Zn²⁺ ions have been reported to have an inhibitory effect on the amidolytic activity of Factor VIIa but no effect on the amidolytic activity of Factor Xa, thrombin or activated Protein C (Pedersen AH et al,*Thromb Haemost* 1991; 65: 528-534). Mn²⁺ ions have also been shown to substitute for calcium ions in the activation of Factor X by Russell Viper Venom enzyme, albeit providing a lower activation rate (Bajaj P et al, *J Biol Chem* 1977; 252: 4758-4761).

The knowledge in the field also teaches that divalent metal ions such as Zn²⁺ and Cu²⁺ stimulate the autoactivation of Factor XII, a non-vitamin K-dependent coagulation factor (Shore et al, *Biochemistry* 1987; 26: 2250-2258; Bernardo MM et al, *J Biol Chem* 1993; 268: 12468-12476).

A further illustration that the present invention was unexpected is the knowledge which teaches that Mg²⁺ and Mn²⁺ stimulate the inhibition of APC by the two plasma protease inhibitors α₂-macroglobulin and plasmin inhibitor (Heeb MJ et al, *J Biol Chem* 1991; 266: 17606-17612). Thus, the addition of Mg²⁺ and Mn²⁺ under the conditions used results in a decreased anticoagulant activity of APC.

Therefore, the present invention providing an increased anticoagulant activity of the Protein C anticoagulant pathway through the use of metal(s) ions such as e.g. divalent metal ions or Cu⁺ in addition to calcium ions, could not be derived or expected from the state-of-the-art knowledge in the field.

### Brief Description of the Drawings and Detailed Description of the Invention

In the following, the invention is disclosed more in detail making reference to the drawings enclosed, wherein:
- **Figure 1**: shows a schematic representation of the blood coagulation system and its regulation;
- **Figure 2**: shows a graphic representation of the results obtained in Example 2, i.e. the effects of the metal ions in a chromogenic Protein C assay;
- **Figure 3**: shows a graphic representation of the effects obtained according to Example 5, namely the effect of Mg²⁺ and Mn²⁺ in a chromogenic Protein S assay; and
- **Figure 4**: assembles in a graphic representation the results obtained according to the method disclosed in Example 8, i.e. the effect of metal ions on discrimination for Protein S deficiency and for FV:Q506 in a global Protein C pathway assay using Factor Xa as activator.

A preferred embodiment of the present invention thus covers a method for the global screening for defects in the Protein C anticoagulant pathway of blood coagulation in a human, comprising
A) incubating a blood sample of said human comprising coagulation factors with:
   1) an activator for the Protein C in said sample,
   2) a suitable coagulation activator,
   3) an exogenous synthetic substrate for either Factor Xa or thrombin comprising a photometrically measurable leaving group,
   4) calcium ions, and
   5) said further metal(s) ions;
B) determining the conversion rate of said exogenous substrate; and
C) comparing said conversion rate with the conversion rate of normal human blood sample determined in the same way.

A further preferred embodiment of the present invention relates to a method for the determination of free Protein S activity, comprising:
A) incubating said blood sample comprising coagulation factors with:
   1) exogenous activated Protein C or exogenous Protein C together with an activator of Protein C,
   2) a suitable coagulation activator,
   3) an exogenous synthetic substrate for either Factor Xa or thrombin comprising a photometrically measurable leaving group,
   4) calcium ions, and
   5) said further metal(s) ions;
B) determining the conversion rate of said exogenous substrate; and
C) comparing said conversion rate with the conversion rate of normal human blood sample determined in the same way.

Another preferred embodiment of the present invention relates to a method for the determination of Protein C activity, comprising:
A) incubating a blood sample of said human comprising coagulation factors with:
   1) an activator for the Protein C in said sample,
   2) a suitable coagulation activator,
   3) an exogenous synthetic substrate for either Factor Xa or thrombin comprising a photometrically measurable leaving group, and
   4) calcium ions, and
   5) said further metal(s) ions;
B) determining the conversion rate of said exogenous substrate; and
C) comparing said conversion rate with the conversion rate of normal human blood sample determined in the same way.

A fourth further preferred embodiment of the present invention covers a method for screening for Factor V mutation(s) in a blood sample of said human, comprising:
A) incubating a blood sample of said human comprising coagulation factors with:
   1) exogenous activated Protein C, or exogenous Protein C together with an activator of Protein C, or an activator for endogenous Protein C,
   2) a suitable coagulation activator,
   3) an exogenous synthetic substrate for either Factor Xa or thrombin comprising a photometrically measurable leaving group,
   4) calcium ions, and
   5) said further metal(s) ions;
B) determining the conversion rate of said exogenous substrate; and
C) comparing said conversion rate with the conversion rate of normal human blood sample determined in the same way.

In the above preferred methods for global screening for defects in the Protein C anticoagulant pathway, for the determination of free Protein S activity or Protein C activity or for screening for Factor V mutation(s) such as the FV:Q506 mutation in a blood sample, step A) comprises incubating the blood sample of said human comprising coagulation factors in the presence of the added further metal(s) ions used according to the present invention with
1) an activator for the Protein C in said sample to provide activation of endogenous Protein C, or exogenous activated Protein C or exogenous Protein C together with an activator of Protein C.
2) a suitable coagulation activator to provide at least partial activation of coagulation,
3) an exogenous synthetic substrate for either Factor Xa or thrombin comprising a photometrically measurable leaving group to provide for monitoring Factor Xₐ or thrombin activity and
4) calcium ions to trigger coagulation.

These steps 1) to 4) can be performed separately and/or simultaneously, i.e. in different sequential combinations providing for so-called "one-stage" to "four-stage" methods as follows:

In a one-stage method all components necessary for performing steps 1) to 4) above are added simultaneously.

In two-stage methods a) the components for steps 1) and 2) may first be combined followed by simultaneous addition of the exogenous substrate with calcium ions (3) and 4)), or b) all the components but for calcium ions (1)-3)) may be added simultaneously, the addition of calcium ions (4) then comprising the second step,or c) the components for step 1), 2) and 4) may be included simultaneously and step 3) be performed as a separate step.

In three-stage methods a) steps 1) and 2) are combined and steps 3) and 4) performed as separate steps, or b) steps 1) and 2) are performed as separate steps and steps 3) and 4) are performed simultaneously, or c) steps 2) and 3) are performed as separate steps, and steps 1) and 4) are performed simultaneously, or d) step 1) is performed as a separate step, steps 2) and 4) are performed simultaneously, followed by step 3).

In four-stage methods steps 1) to 4) are performed as separate steps in the order as described or in any other different order.

All one-, two-, three- or four-stage methods may be used in chromogenic, fluorimetric and luminometric methods.

Other embodiments of the invention comprise clotting methods, utilizing activation through the intrinsic, extrinsic or common pathway.

For any method, said further metal(s) ions used according to the present invention may be added either initially or at a later stage to any of said reagents. In applications where endogenous Protein C is activated by a Protein C activator, such as in methods for Protein C activity and global methods for the Protein C anticoagulant pathway, one preferred mode of the invention is to include said further metal(s) ions in the Protein C activation step, especially when Protac® C is used as the Protein C activator.

Specifically, the invention concerns the addition of divalent metal ions or of the monovalent Cu⁺ ion in view of increasing the resolution between different levels of, respectively, Protein C activity and Protein S activity as well as providing a high discrimination for the presence of the FV:Q⁵⁰⁶ mutation, resulting in an improved sensitivity and specificity for detection of defects in components of the Protein C anticoagulant pathway of blood coagulation. The range of concentrations of metal ions within which the anticoagulant activity of the Protein C system is stimulated varies with respect to the actual metal ion. The optimal concentration for Mg²⁺ ions is considerably higher than that for other metal ions. The concentration range for metal ions other than Mg²⁺ comprises 1 µmol/L - 2mmol/L, suitably 5 - 400 µmol/L and preferably 10 - 80 µmol/L. For Mg²⁺ ions, the concentration range comprises 20 µmol/L - 10 mmol/L, suitably 100 µmol/L - 2 mmol/L and preferably 200 µmol/L - 1 mmol/L.

The counter ion should be selected in such a way to allow the above described available concentrations of metal ions. Suitable counter ions are mono-, di- and trivalent anions, preferably mono- and divalent anions, such as chloride, sulphate and nitrate anions. Metal ions could also be provided in form of a metal ion complex with protein(s) such as blood protein or on a solid surface such as a metal ion coated wall of a reaction vessel.

For any application of the invention, photometric and clotting methods are used for monitoring the anticoagulant activity of components in the Protein C anticoagulant pathway.

In photometric methods, synthetic substrates with colorimetric, fluorimetric or luminometric leaving groups are used, which substrates preferably should be selective for Factor Xa or for thrombin. Since the generation of Factor Xa and thrombin according to the invention is influenced by the Protein C anticoagulant activity in the reaction mixture, containing a test sample from an individual, the measurement of the conversion rate of said substrates by said enzymes is suitably performed for determination of said Protein C anticoagulant activity. The measurement of said conversion rate is suitably compared with the corresponding conversion rate obtained when using a normal human plasma pool as a test sample. The conversion rate may be measured kinetically, i.e. by monitoring the change in optical density (OD) versus time, expressed as e.g. ΔOD/min, or measured after a fixed incubation time, expressed as OD.

The determination of the conversion rate of synthetic substrates is performed with instruments suitable for monitoring the release of the leaving group from the actual substrate chosen. When the conversion rate is determined in a microplate reader, it is suitable to perform readings in the so called dual wavelength mode in order to eliminate possible differences between microplate wells. In such readings, one wavelength is selected for detecting the release of the leaving group whereas the other wavelength is selected in a wavelength range where the released leaving group does not have any appreciable absorbance. When a colorimetric leaving group such as paranitroaniline (pNA) is chosen, a suitable dual wavelength reading is performed at 405 and 490 nanometers, expressed as OD₄₀₅₋₄₉₀ₙₘ.

A further aspect of the invention is the use of diluted blood samples in said photometric methods in order to avoid interference of blodd sample components in the test sample, such as e.g. antibodies against phopholipid-protein complexes (present in e.g. patients with Lupus anticoagulant). The final concentration, i.e. the concentration in the sample the optical density of which is determined, of sample may vary depending on the actual method used. For colorimetric methods said blood sample concentration may be below 10% and preferably below 5%.

Any activator of endogenous Protein C may be used such as thrombin with or without thrombomodulin, both from human or non-human sources or being produced by recombinant technology as wild-type proteins or as modified polypeptides to provide the suitable functional property, or alternatively a snake venom enzyme which activates Protein C. Suitable snake venom enzymes are preferably selected from the Agkistrodon snake family and may be added as crude venom or in a purified state as the product Protac® C. Suitable snake venom enzymes may also be produced by recombinant technology as wild-type proteins or as modified polypeptides to provide the suitable functional property. The concentration of the Protein C activator may vary depending on the actual conditions used. Thus, for the activator Protac®C the concentration may vary between 1 x 10⁻³ and 1 U/mL. preferably 2 x 10⁻³ and 0.3 U/mL during the activation of Protein C in a global method for the Protein C anticoagulant pathway or in specific methods for Protein C and free Protein S activity or for a method for detection of mutations in Factor V/Factor Va which affect the cleavage rate by APC.

According to a further preferred embodiment of the present invention, the activation of Protein C in the blood sample preceeds or occurs simultaneously with activation of coagulation.

In the methods of the present invention, a suitable activator of coagulation is being used. Such activators may be selected to activate the so-called intrinsic, extrinsic or common pathway.

Activation through the intrinsic system may be accomplished with an APTT reagent containing a suitable contact activator, or with the separate addition of a contact activator. As such activators of the intrinsic pathway, suitable compositions of phospholipids and contact activators may be used. Phospholipids may be added as a mixture of purified phospholipids or as crude extracts from biological sources such as e.g. brain, placenta, egg yolk or soybean. As contact activators, ellagic acid, collagen or collagen related substances or various forms of silica (kaolin, micronized silica, colloidal silica) may be used. Alternatively Factor XIIa, Factor XIa or Factor IXa may be used in combination with phospholipids as activators of the intrinsic pathway rather than using a contact activator. Optionally, components such as prothrombin, Factor VIII/Factor VIIIa and Factor X may be added. Photometric substrates selective for Factor Xa or thrombin are used.

Activation through the extrinsic system may be accomplished by tissue factor from human or non-human tissues or produced by recombinant technology as a wild-type protein or as a suitably modified polypeptide with or without the addition of Factor VII/Factor VIIa. Alternatively, activation may be accomplished by Factor VIIa in combination with said phospholipids. Optionally, reagents such as prothrombin, Factor V/Va, Factor IX and Factor X may be added. Photometric substrates selective for FXa or thrombin are used.

Activation of the common pathway may be accomplished by addition of exogenous Factor Xa or by exogenous Factor X in combination with an exogenous activator of Factor X, such as a snake venom enzyme, e.g. a snake venom enzyme from Russeli Viperii. Alternatively, said exogenous activator of Factor X may be added for activation of endogenous Factor X. Optionally, prothrombin and/or Factor V/Va may be added. Photometric substrates selective for thrombin are used.

Generally, interferences due to e.g. variable functional levels of components in the sample may be minimized by including a suitable amount of plasma deficient of the Protein C anticoagulant pathway component to be measured. Such plasmas may be deficient of e.g. Protein C, Protein S or Factor V. In case of a global method for the Protein C anticoagulant pathway, a plasma deficient of Protein C, Protein S and Factor V may be added.

In case of a method for Protein S activity or a method for detection of a Factor V mutation which affects the degradation of Factor V/Va by APC, exogenous Protein C may be added as a plasma deficient of Protein S and Factor V, respectively.

Protein S may be added in methods for Protein C activity or for detection of said mutation(s) in Factor V.

In case of protein C and protein S methods, Factor V/Factor Va may also be added to minimize interference from mutated Factor V present in the sample, vz mutations which affect the degradation of Factor V/Va by APC.

The above-mentioned coagulation factors and components of the Protein C anticoagulant pathway, vz Factor XIIa, Factor XIa, Factor IX/IXa, Factor VIII/VIIIa, Factor VII/VIIa, Factor X/Xa, Factor V/Va, prothrombin, Protein C/APC and Protein S are of human or non-human origin, suitably bovine or human origin. Said coagulation factors may also be produced by recombinant technology as wild-type or as modified polypeptides with suitable biological activity.

In order to prevent fibrin gel formation, a fibrin polymerization inhibitor may be added to the reaction mixture such as Gly-Pro-Arg-Pro.

In chromogenic methods, any chromogenic substrates for Factor Xa may be used, such as e.g. Benzoyl-Ile-Glu-Gly-Arg-pNA (S-2222, Chromogenix AB, Mölndal, Sweden), N-α-Z-D-Arg-Gly-Arg-pNA (S-2765, Chromogenix AB), CH₃SO₂-D-Leu-Gly-Arg-pNA (CBS 31.39, Stago Diagnostica) and MeO-CO-D-CHG-Gly-Arg-pNA (Spectrozyme Xa, American Diagnostica, Greenwich, USA). Correspondingly, any chromogenic substrates for thrombin may be used, e.g. H-D-Phe-Pip-Arg-pNA (S-2238, Chromogenix AB), pyroGlu-Pro-Arg-PNA (S-2366, Chromogenix AB), D-Ala-Pro-Arg-pNA (S-2846, Chromogenix AB), Z-D-Arg-Sarc-Arg-pNA (S-2796, Chromogenix AB), AcOH*H-D-CHG-But-Arg-pNA (CBS 34.47, Stago) and H-D-HHT-Ala-Arg-pNA (Spectrozyme TH, American Diagnostica).

A further subject matter of the present invention is a kit for use in the the above methods comprising the following components:
a) an activator for the Protein C; or exogenous activated Protein C or exogenous Protein C together with an activator of Protein C;
b) a suitable coagulation activator;
c) an exogenous synthetic substrate for either Factor Xa or thrombin comprising a photometrically measurable leaving group;
d) calcium ions; and
e) said further metal(s) ions;
in separate containers and/or in containers comprising mixtures of at least two of said components in aqueous solution or in lyophilized form.

A further preferred embodiment of the present invention relates to a kit for use in the the above methods comprising the following components:
a) an activator for the Protein C; or exogenous activated Protein C or exogenous Protein C together with an activator of Protein C;
b) a suitable coagulation activator;
c) an exogenous synthetic substrate for either Factor Xa or thrombin comprising a photometrically measurable leaving group;
d) calcium ions;
e) said further metal(s) ions;
f) coagulation factors; and
in separate containers and/or in containers comprising mixtures of at least two of said components in aqueous solution or in lyophilized form.

A still further embodiment of the present invention comprises a reagent for use in the above methods comprising at least two of the following components a) to f):
a) an activator for the Protein C; or exogenous activated Protein C or exogenous Protein C together with an activator of Protein C;
b) a suitable coagulation activator;
c) an exogenous synthetic substrate for either Factor Xa or thrombin comprising a photometrically measurable leaving group;
d) calcium ions;
e) said further metal(s) ions; and
f) coagulation factors;
in one container in aqueous solution or in lyophilized form.

One preferred embodiment of said reagent comprises activated Protein C, with or without calcium ions and said further metal(s) ions. One further preferred embodiment of said reagent comprises coagulation factors and-Protein C activators and said further metal(s) ions, if desirable in combination with phospholipid(s). One further embodiment comprises Factor IX/IXa, Factor X/Xa and/or calcium ions and said further metal(s) ions or said further metal(s) ions in combination with Factor V/Va, Protein C and prothrombin. Optionally Factor VIII/VIIIa and/or thrombin may be combined with said further metal(s) ions. In a further embodiment said further metal(s) ions are combined with a Protein C activator, such as Protac® C or thrombin/thrombomodulin. Said reagent embodiments are suitably comprised in a container in aqueous solution or in lyophilized form.

A wide range of concentrations of reactants can be used in the methods of the present invention. The following Table 1 presents suitable and preferred ranges for the various components used in the method and contained in the kits and reagents according to the present invention.

**Table 1**

| **Parameter** | **Final concentration in final reaction medium** |
|---|---|
| Blood sample | 0.02 - 10 %, preferably 0.1-5 % (v/v) |
| FIX/FIXa | 1 x 10 ⁻¹⁵ - 1 x 10 ⁻⁶ mol/L |
| FX/FXa | 1 x 10 ⁻¹⁵ - 5 x 10 ⁻⁷ mol/L |
| FV/FVa | 1 x 10 ⁻¹² - 10 ⁻⁷ mol/L, preferably 2 x 10 ⁻¹⁰ -5 x 10 ⁻⁸ mol/L |
| FVII/FVIIa | 1 x 10 ⁻¹⁵ - 2 x 10 ⁻⁸ mol/L |
| FVIII/FVIIIa | 1 x 10 ⁻⁴ - 5 x 10 ⁻¹ IU/mL |
| Prothrombin | 1 x 10 ⁻⁹ - 5 x 10 ⁻⁷ mol/L |
| Thrombin | 1 x 10 ⁻¹⁵ - 1 x 10 ⁻⁸ mol/L |
| Ca ²⁺ ions | 0.5 ⁻ 20 mmol/L, preferably 1 - 10 mmol/L |
| Mg ²⁺ ions | 20 µmol/L - 10 mmol/L, suitably 100 µmol/L - 2 mmol/L and preferably 200 µmol/L - 1 mmol/L. |
| Mn ²⁺ , Zn ²⁺ , Cu ²⁺ , Ni ²⁺ , Sr ²⁺ and/or Cu ⁺ ions | 1 µmol/L - 2mmol/L, suitably 5 - 400 µmol/L and preferably 10 - 80 µmol/L |
| Protein C/APC | 1 x 10 ⁻¹⁰ - 1 x 10 ⁻⁷ mol/L, preferably 5 x 10 ⁻¹⁰ -1 x 10 ⁻⁸ mol/L |
| Protac®C | 1 x 10 ⁻³ - 1 U/mL, preferably 2 x 10 ⁻³ - 0.3 U/mL |
| Protein S | 10 ⁻⁹ - 5 x 10 ⁻⁷ mol/L |
| Tissue factor | 10 ⁻⁸ - 10 ⁻⁵ g/L, preferably 5 x 10 ⁻⁸ - 10 ⁻⁶ g/L |
| Thrombin/thrombomodulin | 10 ⁻¹¹ - 10 ⁻⁸ mol/L |
| Phospholipid | 1 x 10 ⁻⁶ - 3 x 10 ⁻⁴ mol/L |
| Fibrin polymerization inhibitor | Range dependent of substance used |
| Chromogenic substrate | 10 ⁻⁵ - 5 x 10 ⁻³ mol/L |
| pH | 6.5 - 9.5, preferably 7-8.5 |
| Ionic strength | 0-0.6, preferably 0.01-0.25 |

Suitable embodiments include preparation in aqueous solution or lyophilization in a container of one or more components presented in the above Table 1 such as a protein with and without metal(s) ions, optionally in the presence of phospholipid to provide suitable reagents for use in the method according to the invention. Said embodiments may comprise e.g. Protein C or APC and said further metal(s) ions and with calcium ions and with or without an active enzyme such as Factor IXa or Factor Xa. Other embodiments may comprise metal(s) ions with any or a combination of Factor V/Va, Protein S, prothrombin, Factor X, Factor VIII/VIIIa, thrombin. A further embodiment may comprise a Protein C activator such as Protac® C or thrombin/thrombomodulin with metal(s) ions.

The methods of the present invention allow for convenient reaction times such as 1 - 10 min, preferably 2-5 min, to provide for easy applicability to automated coagulation instruments.

The invention is also concerned with kits and reagents for use in the above in vitro methods for screening and diagnosing for Protein C and Protein S deficiency and for mutations in Factor V which affect the anticoagulant activity of APC. The invention is further concerned with a kit for an in vitro method for screening and diagnosing for defects in the Protein C pathway, caused by e.g. Protein C deficiency, Protein S deficiency and Factor V mutations. Said kits comprise a suitable selection of components listed in Table 1, prefereably present as reagents in one or more container(s) comprising said components in aqueous solution or in lyophilized form.

The invention is illustrated by the following examples which, however, in no way restrict the scope of the claims.

### Example 1

Effect of manganese and magnesium ions on the determination of Protein C activity in a three-stage chromogenic thrombin generation assay using the Protein C activator Protac® C.
Samples: Protein C deficient plasma (Biopool, Umeå, Sweden) with and without addition of purified human Protein C (Chromogenix AB) to yield 0, 0.1, 0.5 and 1.0 IU/mL of Protein C.
Sample dilution: 1:41 in 25 mmol/L Tris-HCl pH 7.6, 20 mmol/L NaCI, 0.2% bovine serum albumin.
Protein C activator: Protac® C was used as a stock solution containing 10 U/mL. Final concentration during activation of Protein C = 0.17 U/mL. Mg²⁺ and Mn²⁺ ions were added to yield final concentrations during activation of Protein C of 0.4 and 0.04 mmol/L, respectively.

| | |
|---|---|
| Reagent 1 | Bovine Factor IXa (Enzyme Research, South Bend, Ill., USA), 180 pmol/L Bovine FX (Chromogenix AB), 0.3 U/mL |
| Reagent 2 | Phospholipids* (Chromogenix AB), 60 µmol/L Gly-Pro-Arg-Pro, 0.36 mg/mL (polymerization inhibitor) Human Factor V, 0.2 U/mL CaCl₂, 6 and 24 mmol/L (final conc. in assay = 1.5 and 6 mmol/L) |

| | |
|---|---|
| *) A mixture of purified phospholipids containing 43% phosphatidylcholine, 27% phosphatidylserine and 30% sphingomyelin. | |

Chromogenic thrombin substrate: S-2796 (Chromogenix AB), 2 mmol/L
Assay in a microplate:

This assay is carried out as a three-stage method comprising, in the first stage, combining 50 µL of the diluted plasma with 50 µl of the Protein C activator Protac®C and incubating this mixture for three minutes at 37°C, whereafter coagulation is activated by adding 50 µL of Reagent 1 and 50 µL of Reagent 2 and incubating the mixture for five minutes at 37°C, whereafter, in the third stage, the substrate hydrolysis is carried out by adding 50 µL of the chromogenic thrombin substrate S-2796 and incubating for four minutes at 37°C. The reaction is then terminated by lowering the pH through addition of 50µL of 20% acetic acid. Thereafter the optical density (OD) of the samples in the microwells is recorded at 405 and 490 nm and the difference in optical density between 405 and 490 nm, OD₄₀₅₋₄₉₀ₙₘ, is calculated. This three-stage reaction is schematically shown as follows:

| | | |
|---|---|---|
| Protein C activation: | Plasma dilution 50 µL | |
| | Protein C activator | 50 µL |
| | *3 min, 37°C* | |
| Coagulation activation | Reagent 1 | 50 µL |
| | Reagent 2 | 50 µL |
| | *5 min, 37°C* | |
| Substrate hydrolysis | S-2796 | 50 µL |
| | *4 min, 37°C* | |
| | HOAc, 20% 50 µL | |
| Recording of OD₄₀₅₋₄₉₀ₙₘ | | |

Results:

| | Protein C, IU/mL | | | |
|---|---|---|---|---|
| | 0 | 0.1 | 0.5 | 1.0 |
| Ca²⁺, 6 mmol/L | 0.542 | 0.515 | 0.503 | 0.467 |
| Ca²⁺, 1.5 mmol/L + Mn²⁺, 0.04 mmol/L | 0.564 | 0.441 | 0.231 | 0.076 |
| Ca²⁺, 1.5 mmol/L + Mg²+, 0.4 mmol/L | 0.541 | 0.441 | 0.230 | 0.069 |

The results demonstrate that by including manganese and magnesium ions in a reaction system containing calcium ions, a strong enhancement of the anticoagulant activity is obtained, manifested by the fact that increasing concentrations of Protein C in the samples result in a much decreased absorbance, i.e. a much decreased thrombin generation. In contrast, in the presence of calcium ions alone, there is a much lower resolution in absorbance, i.e. in thrombin generation, at increasing Protein C concentrations. Thus, the addition of further metal ions constitutes an improved method for determination of Protein C activity.

### Example 2

Effect of different metal ions on determination of Protein C activity in a three-stage thrombin generation assay using a four-fold lower concentration of Protein C activator.

Experimental conditions as in Example 1, except for the use of a final concentration of the Protein C activator (Protac® C) of 0.043 U/mL. Mg²⁺,Mn²⁺, Zn²⁺ and Ni²⁺ ions were added to yield final concentrations during activation of Protein C of 0.4 mmol/L (Mg²⁺) or 0.04 mmol/L. Zn²⁺ ions, Mn²⁺ ions and Cu²⁺ ions were also added to yield a final concentration of 0.08 mmol/L. Ca²⁺ was also used at final concentrations of 1.5 mmol/L and 6.6 mmol/L in the absence of other metal ions.

Results: The results are shown in the table below with all primary data, which also includes a comparison between final concentrations of 0.04 and 0.08 mmol/L for Mn²⁺ and Zn²⁺.

| | Protein C, IU/mL | | | |
|---|---|---|---|---|
| | 0 | 0.1 | 0.5 | 1.0 |
| Ca²⁺, 6 mmol/L | 0.652 | 0.633 | 0.559 | 0.505 |
| Ca²⁺, 1.5 mmol/L | 0.640 | 0.585 | 0.504 | 0.438 |
| Ca²⁺, 1.5 mmol/L + Mn²⁺, 0.04 mmol/L | 0.725 | 0.689 | 0.503 | 0.237 |
| Ca²⁺, 1.5 mmol/L + Mn²⁺, 0.08 mmol/L | 0.627 | 0.469 | 0.145 | 0.056 |
| Ca²⁺, 1.5 mmol/L + Mg²⁺, 0.4 mmol/L | 0.627 | 0.583 | 0.361 | 0.123 |
| Ca²⁺, 1.5 mmol/L + Zn²⁺, 0.04 mmol/L | 0.513 | 0.446 | 0.334 | 0.129 |
| Ca²⁺, 1.5 mmol/L + Zn²⁺, 0.08 mmol/L | 0.421 | - | 0.189 | 0.051 |
| Ca²⁺, 1.5 mmol/L + Ni²⁺, 0.04 mmol/L | 0.594 | 0.437 | 0.173 | 0.047 |
| Ca²⁺, 1.5 mmol/L + Cu²⁺, 0.08 mmol/L | 0.487 | 0.425 | 0.348 | 0.099 |

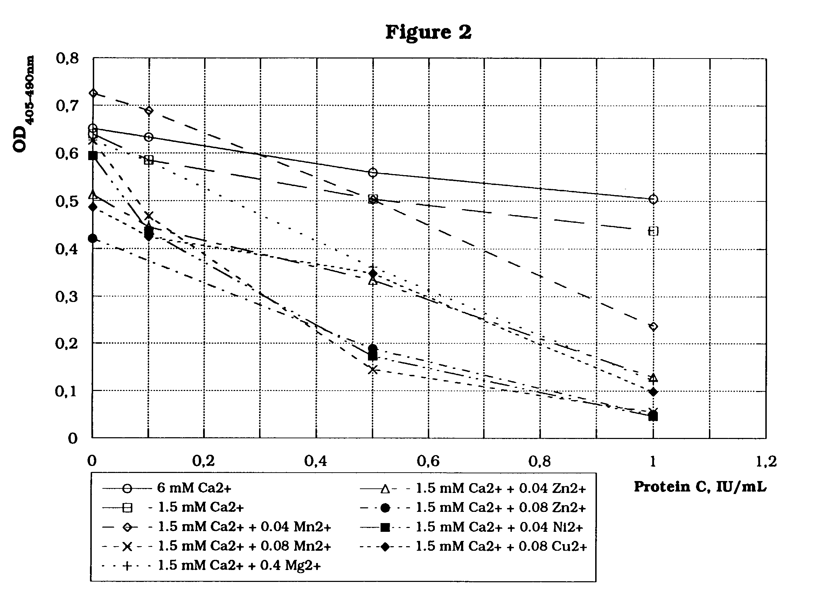

The above results further graphically shown in Figure 2 demonstrate that many different metal ions provide an enhancement of the anticoagulant activity and also that a calcium concentration of 1.5 mmol/L in the absence of any other added further metal ions lacks the anticoagulation enhancement property. Furthermore, the concentration of Protac® C is not critical since the use of a four-fold lower concentration of this component still results in a pronounced anticoagulant activity in the presence of added metal ions.

### Example 3

Effect of metal ions on determination of Protein C activity in a two-stage thrombin generation assay.

These experimental details are as described in Example 1, with the following exceptions:
- the final concentration of the Protein C activator (Protac® C) was 0.043 U/mL during activation of Protein C.
- the chromogenic thrombin substrate used was S-2846 (Chromogenix AB),
- the chromogenic substrate was included in Reagent 1,
- purified human Protein C was added to Protein C deficiency plasma to yield 0, 0.1 and 0.5 IU/mL,
- metal ions tested: Mn²⁺ and Mg²⁺.

Results, expressed as OD₄₀₅₋₄₉₀ₙₘ:

| a) Mn ²⁺ ions | Protein C, IU/mL | | |
|---|---|---|---|
| | 0 | 0.1 | 0.5 |
| Ca ²⁺ , 6 mmol/L | 1.18 | 1.07 | 0.669 |
| Ca ²⁺ , 1.5 mmol/L + Mn ²⁺ , 0.04 mmol/L | 1.36 | 1.17 | 0.258 |

| b) Mg ²⁺ ions | Protein C, IU/mL | | |
|---|---|---|---|
| | 0 | 0.1 | 0.5 |
| Ca ²⁺ , 6 mmol/L | 1.24 | 1.05 | 0.554 |
| Ca ²⁺ , 1.5 mmol/L + Mg ²⁺ , 0.4 mmol/L | 1.45 | 1.15 | 0.215 |

These results show that a significantly higher resolution for the different Protein C activities is obtained when Mn²⁺ and Mg²⁺ ions are added to a final concentration of 0.04 mmol/L and 0.4 mmol/L, respectively, thus constituting an improved two-stage method for determination of Protein C activity.

### Example 4

Effect of manganese ions on determination of Protein C activity in a two-stage thrombin generation assay using a phospholipid emulsion from bovine brain.
Phospholipid source: Cephotest (Nycomed, Oslo, Norway)
Experimental details as in Example 3. The final concentration of Cephotest was 3% (v/v).

Results, expressed as OD₄₀₅₋₄₉₀ₙₘ:

| | Protein C, IU/mL | | |
|---|---|---|---|
| | 0 | 0.1 | 0.5 |
| Ca²⁺, 6 mmol/L | 1.09 | 0.813 | 0.375 |
| Ca²⁺, 1.5 mmol/L + Mn²⁺, 0.04 mmol/L | 1.366 | 0.996 | 0.163 |

The results show that the same enhancing effect on the Protein C anticoagulant activity is obtained with a crude phospholipid extract from an animal tissue source. Hence, the source of phospholipid is not critical.

### Example 5

Effect of manganese and magnesium ions on determination of free Protein S activity in a chromogenic Factor Xa generation assay.
Sample: Protein S deficient plasma (Biopool) with or without addition of purified human Protein S to yield 0%, 25% and 100% Protein S.
Sample dilution: 1:61 in 50 mmol/L Tris buffer pH 8.2, 0.15 mol/L Nacl, 0.2% BSA
Factor reagent (concentration in assay before substrate addition):
Bovine FIXa (4 mU/mL)
Bovine FX (0.3 U/mL)
Human FVIII (0.02 U/mL)
Human FV (0.02 U/mL)
Human prothrombin (0.01 U/mL)
Phospholipids (21 µmol/L)
Mg²⁺ (0.4 mmol/L) or Mn²⁺ (0.04 mmol/L) or no addition
Medium: 10 mmol/L MES pH 6.0, 0.15 mol/L NaCl, 0.2% BSA
Start reagent
Human APC (0.35 µg/mL)
CaCl₂ (1.5 mmol/L)

### Chromogenix Factor Xa substrate: S-2765 (Chromogenix AB), 1.8 mmol/L.

For carrying out the assay 50 µL of the diluted plasma sample was mixed with 50 µL of the above Factor reagent, whereafter the mixture was incubated for three minutes at 37°C. Thereafter, 50 µL of the Start reagent comprising human APC and calcium chloride was added and the mixture incubated for four minutes at 37°C. Following that, 50 µL of the chromogenic substrate S-2765 was added to and the reaction mixture incubated for two minutes at 37°C, whereafter 50 µL acetic acid was added to terminate the reaction. The absorbance of the sample was then determined according to Example 1 and expressed as OD_{405-490 nm}.

| | | |
|---|---|---|
| Assay | Factor reagent | 50 µL |
| | Sample dilution | 50 µL |
| | *Incubation 3 min, 37°C* | |
| | APC/CaCl₂ | 50 µL |
| | *4 min, 37°C* | |
| | S-2765, 1.8 mmol/L | 50 µL |
| | *2 min, 37°C* | |
| | HOAc, 20% | 50 µL |

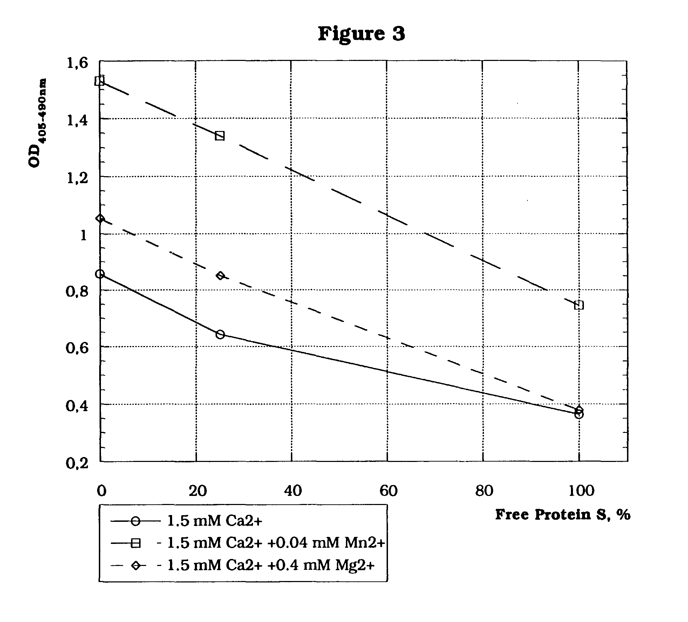

Results: All primary data are listed in the table below and also illustrated in Figure 3.

| | Free Protein S, % | | |
|---|---|---|---|
| | 0 | 25 | 100 |
| Ca²⁺, 1.5 mmol/L | 0.857 | 0.642 | 0.364 |
| Ca²⁺, 1.5 mmol/L + Mn²⁺, 0.04 mmol/L | 1.53 | 1.34 | 0.745 |
| Ca²⁺, 1.5 mmol/L + Mg²⁺, 0.4 mmol/L | 1.053 | 0.851 | 0.378 |

Figure 3 shows that the addition of Mg²⁺ or Mn²⁺ ions results in a greater resolution, (i.e. a greater slope of the curve) as well as in a more linear dose response when compared to the use of Ca²⁺ alone, thus constituting an improved method for determination of Protein S activity.

### Example 6

Effect of strontium ions on the determination of free Protein S activity in a chromogenic Factor Xa generation assay.

The experimental details are as disclosed in Example 5 but with the Factor reagent stored for one hour before assay.

Results: Absorbances expressed as OD₄₀₅₋₄₉₀ₙₘ

| | Free Protein S, % | | |
|---|---|---|---|
| | 0 | 25 | 100 |
| Ca²⁺, 1.5 mmol/L | 0.673 | 0.488 | 0.258 |
| Ca²⁺, 1.5 mmol/L + Sr²⁺, 0.4 mmol/L | 0.848 | 0.611 | 0.276 |

The results show that a higher resolution for various Protein S activity levels is obtained on addition of Sr²⁺ ions, supporting the enhancing effect of Sr²⁺ on the Protein C anticoagulant pathway activity.

### Example 7

Effect of metal ions on the detection of Protein S deficiency in a global chromogenic method for the Protein C anticoagulant pathway, using tissue factor as activator of coagulation and monitoring thrombin generation.
Sample: Normal human plasma pool and Protein S deficient plasma (Biopool, Umeå, Sweden)
Sample dilution: 1:21 in 25 mmol/L Tris-HCl pH 7.6, 20 mmol/L NaCl, 0.2% bovine serum albumin.
Protein C activator:Protein C activator (Protac® C) from Coamatic Protein C, was reconstituted in 7.2 mL according to the kit package insert and then diluted in 25 mmol/L Tris-HCl pH 7.6, 20 mmol/L NaCl, 0.2% bovine serum albumin to yield a concentration during Protein C activation of 0.02 U/mL. Human prothrombin (Chromogenix AB) was added to yield a final concentration after addition of tissue factor of 1.5 µg/mL.

The analysis was performed with or without Mg²⁺ ions added to the Protac® C solution.

### Reagent:

Tissue factor: Thromborel (Behringwerke, Marburg, Germany). Reconstituted in 2 mLwater according to the manufacturer, thereafter diluted in 25 mmol/L Tris-HCl pH 7.6, 20 mmol/L NaCl, 0.2% bovine serum albumin to yield a final concentration during activation of coagulation of 0.033% (v/v).
Phospholipids (43% phosphatidylcholine, 27% phosphatidylserine and 30% sphingomyelin): Final concentration during activation of coagulation of 16.7 µmol/L.
CaCl₂: 6.6 mmol/L final concentration during activation of coagulation.
Chromogenic thrombin substrate: S-2796 (Chromogenix AB), 1.8 mmol/L

For carrying out the assay 50 µL of the diluted plasma sample was mixed with 50 µL of the Protein C activator, whereafter the mixture was incubated for two minutes at 37°C. Thereafter, 50 µL of the reagent comprising the tissue factor was added and the mixture incubated for two minutes at 37°C. Following that, 50 µL of the chromogenic substrate S-2796 was added and the reaction mixture incubated for four minutes at 37°C, whereafter 50 µL acetic acid solution was added to terminate the reaction. The absorbance of the sample was then determined according to Example 1 and expressed as OD_{405-490 nm}.

| | | |
|---|---|---|
| Microplate Assay | | |
| | Sample dilution | 50 µL |
| | Protac C activator | 50 µL |
| | *2 min, 37°C* | |
| | Reagent | 50 µL |
| | *2 min, 37°C* | |
| | S-2796 | 50 µL |
| | *4 min, 37°C* | |
| | HOAc, 20 % | 50 µL |

### Results:

| | Normal plasma | Protein S def. plasma |
|---|---|---|
| Ca ²⁺ , 6.6 mmol/L | 0.26 | 0.53 |
| Ca ²⁺ , 6.6 mmol/L + Mg ²⁺ , 0.4 mmol/L | 0.29 | 0.75 |

The results show that the addition of magnesium ions to calcium ions brings about a higher resolution at different Protein S activity levels thus improving detection of Protein S deficiency.

### Example 8

Effect of metal ions on resolution between different levels of free Protein S and for detection of FV:Q506 in a global method for the Protein C anticoagulant pathway using Factor Xa as activator of coagulation and monitoring thrombin generation.
Experimental details as in Example 7, but with bovine Factor Xa (Chromogenix AB) used instead of tissue factor as activator of coagulation. Concentration of Factor Xa = 1.4 ng/mL during activation. Furthermore, a stock solution of Protac® C, containing 10 U/mL, was used, which was then diluted in 25 mmol/L Tris-HCl pH 8.4, 0.2% bovine serum albumin to yield a concentration during protein C activation of 0.02 U/mL.

| | | |
|---|---|---|
| Samples | 100% protein S = | normal human plasma pool |
| | 0% protein S = | protein S deficient plasma |
| | 25% protein S = | protein S deficient plasma + 2.5 µg/mL purified human protein S. |

Furthermore, a sample from an individual with heterozygosity for the factor V mutation (FV:R506Q) was analysed.

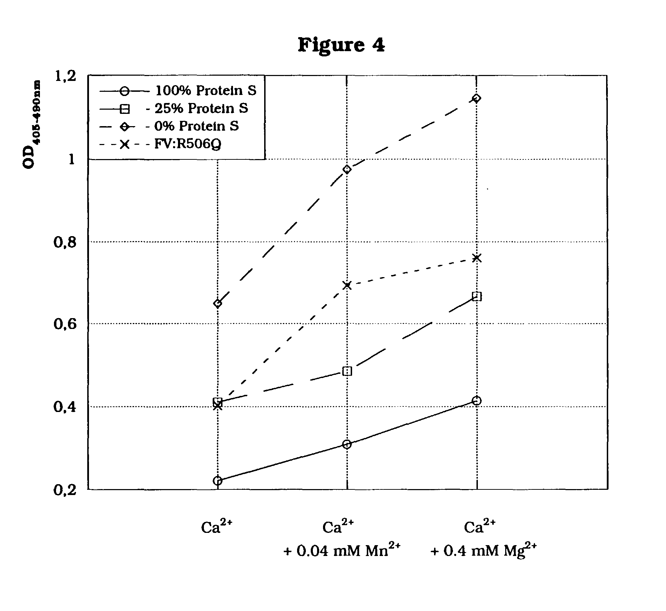

Results: See Figure 4. The table below presents all primary data expressed as OD₄₀₅₋₄₉₀ₙₘ.

| Sample | Only Ca²⁺ | Ca²⁺ + 0.04 mM Mn²⁺ | Ca²⁺ + 0.4 mM Mg²⁺ |
|---|---|---|---|
| 100% Protein S | 0.222 | 0.309 | 0.414 |
| 25% Protein S | 0.411 | 0.485 | 0.667 |
| 0% Protein S | 0.650 | 0.975 | 1.147 |
| FV:R506Q | 0.402 | 0.693 | 0.761 |

The results show that a higher resolution is obtained for Protein S deficiency in the 0 - 100% range as well as a high discrimination for the FV:Q506 mutation when Mg²⁺ or Mn²⁺ ions are included in the reaction mixture, thus proving the beneficial use of added metal ions in a global chromogenic method.

### Example 9

Comparison between a global chromogenic method according to the invention , using Factor Xa as coagulation activator, with a global clotting method according to the prior art using APTT as coagulation activator, regarding resolution between different levels of, respectively, Protein C and Protein S activity and regarding analysis of plasma from pregnant women.

Samples: Human normal plasma pool (NPL), three plasmas from healthy individuals (N1-N3), four plasmas from pregnant women (P1-P4) and plasmas with 0% and 50% deficiency of Protein C and Protein S, respectively, the 50% levels being prepared by adding purified human Protein C (Chromogenix AB) or Protein S (Chromogenix AB) to plasmas deficient in either Protein C or S (both from Biopool AB).

Global chromogenic assay: Experimental details and assay, see Examples 7 and 8. A stock solution of Protac® C, containing 10 U/mL, was used which was then diluted to yield a final concentration during activation of Protein C = 0.02 U/mL. Metal ion used = Mn²⁺, which was added to the Protac® C solution to yield 0.04 mmol/L in the Protein C activation step. The analysis was performed in a microplate and the OD₄₀₅₋₄₉₀ₙₘ was determined as described in Example 1. A high OD₄₀₅₋₄₉₀ corresponds to pronounced thrombin formation and thus an impaired Protein C anticoagulant pathway activity.

Global clotting assay using APTT reagent: APTT reagent from Coatest® APC Resistance was used at a final phospholipid concentration of 33 µmol/L during coagulation activation. For activation of Protein C, the same Protac® C stock solution and dilution medium was used as for the chromogenic assay. Final concentration during activation of Protein C = 0.083 U/mL. The analysis was performed in a ST-4 coagulation analyzer (Diagnostica Stago).

| | | |
|---|---|---|
| Assay | Plasma sample | 50 µL |
| | Protac®C or buffer | 50 µL |
| | APTT reagent | 50 µL |
| | Activation for 3 min, 37°C | |
| | Ca²⁺, 25 mmol/L | 50 µL |

The clotting time in seconds was determined in the presence (CT+) and absence (CT-) of Protac® C and a clot time ratio (CTR) was calculated as CTR = CT+/CT-. A low CTR corresponds to a pronounced thrombin formation also in the presence of Protac® C and hence an impaired Protein C anticoagulant pathway activity.

Results:

| Sample | Chromogenic | APTT |
|---|---|---|
| | OD₄₀₅₋₄₉₀ | CTR |
| NPL | 0.202 | 3.77 |
| N1 | 0.183 | 5.17 |
| N2 | 0.182 | 3.50 |
| N3 | 0.186 | 4.85 |
| P1 | 0.221 | 3.15 |
| P2 | 0.298 | 2.21 |
| P3 | 0.239 | 2.60 |
| P4 | 0.259 | 2.66 |
| 50% Pr S | 0.578 | 3.48 |
| 0% Pr S | 0.802 | 1.80 |
| 50% Pr C | 0.456 | 3.86 |
| 0% Pr C | 1.084 | 1.18 |

The results demonstrate that a) for samples with 50% deficiency of either Protein C or Protein S a higher resolution versus the normal samples and b) for samples from pregnant women a smaller deviation from normal samples is obtained with the chromogenic assay, thus supporting that a higher sensitivity and specificity will be obtained with a global chromogenic assay according to the invention as compared to a global clotting method according to the prior art.

### Example 10

Effect of a mixture of Mg²⁺ and Mn²⁺ in a phospholipid reagent or in an APC reagent on discrimination for the FV:Q506 mutation in a chromogenic thrombin generation assay using Factor Xa as activator.
Sample: Plasma with normal factor V (R506R) and with hetero-(R506Q) and homozygosity (Q506Q) for FV:Q506 mutation.
- Sample dilution:: 1:41 in 0.05 mol/L Hepes pH 7.7, 0.15 mol/L NaCl.

| | |
|---|---|
| Reagent A | Human prothrombin, 19 µg/mL Phospholipids (43% phosphatidylcholine. 27% phosphatidylserine and 30% sphingomyelin), 50 µmol/L |
| Reagent B | Bovine Factor Xa, 0.2 nmol/L APC, 6 µg/mL CaCl₂, 25 mmol/L |

Chromogenic thrombin substrate: S-2796 (Chromogenix AB), 1.8 mmol/L
Mixture of metal ions: Mg²⁺ 0.4 mmol/L, and Mn²⁺,0.04 mmol/L included in either Reagent A or Reagent B.

For carrying out the assay 50 µL of Reagent A was mixed with 50 µL of Reagent B, whereafter the mixture was incubated for three minutes at 37°C. Thereafter, 50 µL of the plasma dilution was added and incubated for two minutes at 37°C. Following that, 50 µL of the chromogenic substrate S-2796 was added and kinetic reading was performed. The change in OD₄₀₅₋ 490 per minute was determined and expressed as ΔOD₄₀₅₋₄₉₀/min.

| | | |
|---|---|---|
| Assay | Reagent A | 50 µL |
| | Reagent B | 50 µL |
| | *Incubate at 37°C for 3 min* | |
| | Plasma dilution | 50 µL |
| | *2 min, 37°C* | |
| | S-2796 | 50 µL |
| | Kinetic reading | |

### Results:

| | Mg²⁺ and Mn²⁺ in Reagent A | Mg²⁺ and Mn²⁺ in Reagent B |
|---|---|---|
| FV:R506R | 0.143 | 0.193 |
| FV:R506Q | 0.646 | 0.616 |
| FV:Q506Q | 1.116 | 0.942 |

The results show that a mixture of metal ions such as Mg²⁺ and Mn²⁺ may be added in a phospholipids containing reagent (Reagent A) or in a reagent containing active enzymes such as APC and Factor Xa (Reagent B)and provide a high discrimination for the FV:Q506 mutation. Hence the addition of further metal(s) ions is not restricted to any unique reagent.

### Example 11

Substitution of chloride anions with nitrate and sulfate anions in a study on the effect of magnesium and manganese in determination of Protein C activity in a three-stage thrombin generation assay.

### Experimental details as in Example 1.

Magnesium nitrate (Mg(NO₃)₂)] and manganese sulfate (MnSO₄) were used instead of the corresponding chloride salts at final concentrations in the assay of 0.4 and 0.04 mmol/L respectively in accordance with the conditions in Example 1. Absorbance readings are expressed as OD₄₀₅₋₄₉₀ₙₘ.

| Results: | Protein C, IU/mL | | | |
|---|---|---|---|---|
| | 0 | 0.1 | 0.5 | 1.0 |
| Ca²⁺, 1.5 mmol/L + Mn²⁺, 0.04 mmol/L | 0.563 | 0.541 | 0.278 | 0.079 |
| Ca²⁺, 1.5 mmol/L + Mg²⁺, 0.4 mmol/L | 0.603 | 0.554 | 0.448 | 0.154 |

The results show that a similar high resolution is obtained as when using chloride as an anion (cf. Example 1). Thus, the choice of the anion is not restricted to chloride ions.

## Claims

1. In vitro photometric method for qualitative screening and quantitative determination of the functional activity of components of the Protein C anticoagulant pathway of blood coagulation, comprising measuring the conversion rate of an exogenous substrate by an enzyme, the activity of which is related to the Protein C anticoagulant activity, in a blood sample of a human comprising coagulation factors and said exogenous substrate after at least partial activation of coagulation through the intrinsic, extrinsic or common pathway and triggering coagulation by adding calcium ions; and comparing said conversion rate with the conversion rate of a normal human blood sample determined in the same way, **characterized by** adding further metal(s) ions to said sample.

2. The method according to claim 1, **characterized in that** divalent metal ions or monovalent copper ions are used as said further metal(s) ions.

3. The method according to claim 1 or 2, **characterized by** using Mg²⁺, Mn²⁺, Zn²⁺, Cu²⁺, Ni²⁺, Sr²⁺, and/or Cu⁺ ions as said further metal(s) ions.

4. The method according to claims 1 to 3 for the global screening for defects in the Protein C anticoagulant pathway of blood coagulation in a human, comprising:
A) incubating a blood sample of said human comprising coagulation factors with:
1) an activator for the Protein C in said sample,
2) a suitable coagulation activator,
3) an exogenous synthetic substrate for either Factor Xa or thrombin comprising a photometrically measurable leaving group,
4) calcium ions, and
5) said further metal(s) ions;
B) determining the conversion rate of said exogenous substrate; and
C) comparing said conversion rate with the conversion rate of normal human blood sample determined in the same way.

5. The method according to claims 1 to 3 for the determination of free Protein S activity in a blood sample of said human, comprising:
A) incubating said blood sample comprising coagulation factors with:
1) exogenous activated Protein C or exogenous Protein C together with an activator of Protein C,
2) a suitable coagulation activator,
3) an exogenous synthetic substrate for either Factor Xa or thrombin comprising a photometrically measurable leaving group,
4) calcium ions, and
5) said further metal(s) ions;
B) determining the conversion rate of said exogenous substrate; and
C) comparing said conversion rate with the conversion rate of normal human blood sample determined in the same way.

6. The method according to claims 1 to 3 for the determination of Protein C activity in a blood sample of said human, comprising:
A) incubating a blood sample of said human comprising coagulation factors with:
1) an activator for the Protein C in said sample,
2) a suitable coagulation activator,
3) an exogenous synthetic substrate for either Factor Xa or thrombin comprising a photometrically measurable leaving group,
4) calcium ions, and
5) said further metal(s) ions;
B) determining the conversion rate of said exogenous substrate; and
C) comparing said conversion rate with the conversion rate of normal human blood sample determined in the same way.

7. The method according to claims 1 to 3 for screening for Factor V mutation(s) in a blood sample of said human, comprising:
A) incubating a blood sample of said human comprising coagulation factors with:
1) exogenous activated Protein C, or exogenous Protein C together with an activator of Protein C, or an activator for endogenous Protein C,
2) a suitable coagulation activator,
3) an exogenous synthetic substrate for either Factor Xa or thrombin comprising a photometrically measurable leaving group,
4) calcium ions, and
5) said further metal(s) ions;
B) determining the conversion rate of said exogenous substrate; and
C) comparing said conversion rate with the conversion rate of normal human blood sample determined in the same way.

8. The method according to any one of the preceding claims, **characterized in that** the blood sample is blood or a blood derived sample such as a blood plasma sample or a blood serum sample.

9. The method according to any one of claims 4 to 8, **characterized in that** said stages 1) to 5) of incubation step A, i.e. 1) addition of an activator for endogenous Protein C, or of exogenous activated Protein C, or of exogenous Protein C together with an activator of Protein C, 2) addition of a suitable coagulation activator to provide at least partial activation of coagulation, 3) addition of an exogenous synthetic substrate for either Factor Xa or thrombin 4) addition of calcium ions, and 5) addition of said further metal(s) ions respectively, can be performed separately and/or simultaneously.

10. The method according to any one of the preceding claims, **characterized by** adding said further metal(s) ions in the Protein C activation stage .

11. The method according to any one of the preceding claims, **characterized in that** said calcium ions are used in a concentration of 0.5 to 20 mmol/L, preferably 1 to 10 mmol/L of the final assay medium.

12. The method according to any one of the preceding claims, **charac** **terized in that** said Mg²⁺ ions are used in a concentration of 20 µmol/L to 10 mmol/L, preferably 100 µmol/L to 2 mmol/L and, more preferably 200 µmol/L to 1 mmol/L of the final assay medium.

13. The method according to any one of the preceding claims, **characterized in that** said Mn²⁺, Zn²⁺, Cu²⁺, Ni²⁺, Sr²⁺, and/or Cu⁺ ions are used in a concentration of 1 µmol/L - 2mmol/L, preferably 5 to 400 µmol/L and, more preferably 10 to 80 µmol/L of the final assay medium.

14. The method according to any one of the preceding claims, **characterized in that** the activation of Protein C in said sample precedes or occurs simultaneously with activation of coagulation.

15. The method according to any one of the preceding claims, **characterized in that** an activator for Protein C selected from the group comprising Protein C activating snake venom enzymes and thrombin, if desired thrombin in combination with thrombomodulin, is used.

16. The method according to claim 15, **characterized in that** a Protein C activating snake venom enzyme obtained from the Agkistrodon family of snakes, preferably from *Agkistrodon contortrix contortrix* is used.

17. The method according to claim 16, **characterized in that** the crude venom or the snake venom enzyme preparation such as a snake venom enzyme selected from the Agkistrodon snake family (Protac® C) is used.

18. The method according to claim 17, **characterized by** using the Protein C activator such as a snake venom enzyme selected from the Agkistrodon snake family (Protac® C) in an amount of 1x10⁻³ to 1 U/mL, preferably 2x10⁻³ to 0.3 U/mL in the final assay medium.

19. The method according to any one of the preceding claims, **characterized by** using as suitable coagulation activators for the intrinsic pathway compositions comprising
a) phospholipid(s) and
b) contact activators and/or activated Factors IX, XII or XI or a reagent which generates activated factors IX, XII or XI in vitro.

20. The method according to claim 19, **characterized by** using as the phospholipid(s) a purified phospholipid or a mixture thereof, or crude extracts of animal tissues, extracts from brain, placenta, egg yolk or from soybeans.

21. The method according to claims 19 and 20, **characterized by** using a contact activator selected from the group comprising ellagic acid, collagen, collagen related substances or a silica, such as micronized silica, colloidal silica and kaolin.

22. The method according to any one of the preceding claims, **characterized by** using native or recombinant human or non-human tissue factor (thromboplastin) from human or non-human species with or without Factor VII/VIIa, or (native or recombinant human or non-human) Factor VIIa and phospholipids as a suitable coagulation activator for the extrinsic pathway.

23. The method according to any one of the preceding claims, **characterized by** using exogenous activated Factor X, or exogenous Factor X and an exogenous activator for Factor X, or an exogenous activator for endogenous Factor X as a suitable coagulation activator for the common pathway.

24. The method according to claim 23, **characterized by** using a snake venom enzyme from Russelli Viperii as an exogenous activator for Factor X.

25. The method according to any one of the preceding claims, **characterized in that** components of the Protein C anticoagulant pathway are added to the reaction medium to compensate for variable functional levels of such components in the sample.

26. The method according to claim 25, **characterized by** using components of the Protein C anticoagulant pathway selected from the group comprising Protein C, activated Protein C, Protein S, Factor V/Factor Va, or a plasma deficient of the actual Protein C anticoagulant pathway component to be measured or a plasma deficient of all said components of the Protein C anticoagulant pathway.

27. The method according to any one of the preceding claims, **characterized in that** a fibrin polymerization inhibitor, such as Gly-Pro-Arg-Pro is added to the reaction medium.

28. The method according to any one of the preceding claims, **characterized in that** coagulation factors selected from the group comprising Factor VIII/Factor VIIIa, Factor IX, Factor X and prothrombin are added to the reaction medium.

29. The method according to any one of the preceding claims, **characterized in that** the coagulation factors used are selected from human or non-human sources or being produced by recombinant technology as wild-type proteins or as modified polypeptides to provide the suitable functional property.

30. The method according to any one of the preceding claims, **characterized by** using an exogenous synthetic substrate for either Factor Xa or thrombin in the reaction mixture.

31. The method according to claim 30, **characterized in that** as the exogenous synthetic substrate for either Factor Xa or thrombin a photometric substrate comprising a chromophore, a fluorophore or a luminophore as a leaving group is used.

32. The method according to claim 31, **characterized in that** a photometric substrate comprising a p-nitroaniline group (pNA) as a chromophoric leaving group, a naphthylamine or coumarine derivative group as a fluorophoric leaving group, and an isoluminolamide group as a luminophoric leaving group.

33. The method according to claim 30 to 32, **characterized in that** as substrate for Factor Xa Benzoyl-Ile-Glu-Gly-Arg-pNA (S-2222), N-a-Z-D-Arg-Gly-Arg-pNA (S-2765), CH₃SO₂-D-Leu-Gly-Arg-pNA (CBS 31.39) or MeO-CO-D-CHG-Gly-Arg-pNA (Spectrozyme Xa) is used.

34. The method according to claim 30 to 32, **characterized in that** as a substrate for thrombin H-D-Phe-Pip-Arg-pNA (S-2238), pyroGlu-Pro-Arg-pNA (S-2366), H-D-Ala-Pro-Arg-pNA (S-2846), Z-D-Arg-Sarc-Arg-pNA (S-2796), AcOH*H-D-CHG-But-Arg-pNA CBS 34.47) or H-D-HHT-Ala-Arg-pNA (Spectrozyme TH) is used.

35. A kit for use in the methods according to any one of the preceding claims comprising the following components:
a) an activator for the Protein C; or exogenous activated Protein C or exogenous Protein C together with an activator of Protein C;
b) a suitable coagulation activator;
c) an exogenous synthetic substrate for either Factor Xa or thrombin comprising a photometrically measurable leaving group;
d) calcium ions; and
e) said further metal(s) ions; in separate containers and/or in containers comprising mixtures of at least two of said components in aqueous solution or in lyophilized form.

36. The kit according to claim 35 characterized by additionally comprising f) coagulation factors.

37. A reagent for use in the methods according to any one of claims 1 to 34, comprising at least two of the following components a) to f):
a) an activator for the Protein C; or exogenous activated Protein C or exogenous Protein C together with an activator of Protein C;
b) a suitable coagulation activator;
c) an exogenous synthetic substrate for either Factor Xa or thrombin comprising a photometrically measurable leaving group;
d) calcium ions;
e) said further metal(s) ions; and
f) coagulation factors;
**characterized by** comprising said further metal(s) ions in combination with a Protein C activator, such as a snake venom enzyme selected from the Agkistrodon snake family (Protac® C) or thrombin/thrombomodulin, in one container in aqueous solution or in lyophilized form.

## Patentansprüche

1. Photometrisches in vitro-Verfahren zur qualitativen Überprüfung und quantitativen Bestimmung der funktionellen Aktivität von Bestandteilen des antikoagulatorischen Protein C-Wegs der Blutgerinnung, umfassend die Bestimmung der Umwandlungsrate eines exogenen Substrats durch ein Enzym, dessen Aktivität mit der antikoagulatorischen Aktivität von Protein C verknüpft ist, in einer Blutprobe eines Menschen, die Gerinnungsfaktoren und das exogene Substrat enthält, nach mindestens teilweiser Aktivierung der Gerinnung durch den intrinsischen-, extrinsischen- oder gemeinsamen Weg und Auslösen der Gerinnung durch Zugabe von Calciumionen: und Vergleichen der Umwandlungsrate mit der Umwandlungsrate einer normalen menschlichen Blutprobe, die in gleicher Weise bestimmt wird, gekennzeichnet durch die Zugabe von Ionen eines oder mehrerer weiterer Metalle (weitere Metallionen) zu der Probe.

2. Verfahren nach Anspruch 1, dadurch gekennzeichnet, daß zweiwertige Metallionen oder einwertige Kupferionen als die weiteren Metallionen verwendet werden.

3. Verfahren nach Anspruch 1 oder 2, gekennzeichnet durch die Verwendung von Mg²⁺-, Mn²⁺-, Zn²⁺-, Cu²⁺-, Ni²⁺-, Sr²⁺- und/oder Cu⁺-Ionen als die weiteren Metallionen.

4. Verfahren nach den Ansprüchen 1 bis 3 für die globale Überprüfung von Defekten in dem antikoagulatorischen Protein C-Weg der Blutgerinnung eines Menschen, umfassend:
A) Inkubieren einer Blutprobe des Menschen, die Gerinnungsfaktoren umfaßt mit:
1) einem Aktivator für das in der Probe vorhandene Protein C.
2) einem geeigneten Gerinnungsaktivator,
3) einem exogenen synthetischen Substrat für entweder Faktor Xa oder Thrombin, welches eine photometrisch meßbare austretende Gruppe aufweist.
4) Calclumionen und
5) die weiteren Metallionen;
B) Bestimmung der Umwandlungsrate des exogenen Substrats; und
C) Vergleich der Umwandlungsrate mit der Umwandlungsrate der normalen menschlichen Blutprobe, die in gleicher Weise bestimmt worden ist.

5. Verfahren nach den Ansprüchen 1 bis 3 zur Bestimmung der Aktivität von freiem. Protein S in einer Blutprobe des Menschen, umfassend:
A) Inkubieren der Blutprobe, die Gerinnungsfaktoren umfaßt, mit:
1) exogenem aktiviertem Protein C oder exogenem Protein C zusammen mit einem Aktivator für Protein C,
2) einem geeigneten Gerinnungsaktivator,
3) einem exogenen synthetischen Substrat für entweder Faktor Xa oder Thrombin, welches eine photometrisch meßbare austretende Gruppe aufweist,
4) Calciumionen und
5) die weiteren Metallionen;
B) Bestimmung der Umwandlungsrate des exogenen Substrats; und
C) Vergleich der Umwandlungsrate mit der Umwandlungsrate der normalen menschlichen Blutprobe, die in gleicher Weise bestimmt worden ist.

6. Verfahren nach den Ansprüchen 1 bis 3 zur Bestimmung der Protein C-Aktivität in einer Blutprobe des Menschen, umfassend:
A) Inkubieren einer Blutprobe des Menschen, die Gerinnungsfaktoren umfaßt, mit:
1) einem Aktivator für das in der Probe vorhandene Protein C,
2) einem geeigneten Gerinnungsaktivator,
3) einem exogenen synthetischen Substrat für entweder Faktor Xa oder Thrombin, welches eine photometrisch meßbare austretende Gruppe aufweist,
4) Calciumionen und
5) die weiteren Metallionen;
B) Bestimmung der Umwandlungsrate des exogenen Substrats; und
C) Vergleich der Umwandlungsrate mit der Umwandlungsrate der normalen menschlichen Blutprobe, die in gleicher Weise bestimmt worden ist.

7. Verfahren nach den Ansprüchen 1 bis 3 zur Überprüfung von Faktor V-Mutation(en) in einer Blutprobe des Menschen, umfassend:
A) Inkubieren der Blutprobe des Menschen, die Gerinnungsfaktoren umfaßt, mit:
1) exogenem aktiviertem Protein C oder exogenem Protein C zusammen mit einem Aktivator für Protein C, oder einem Aktivator für endogenes Protein C,
2) einem geeigneten Gerinnungsaktivator,
3) einem exogenen synthetischen Substrat für entweder Faktor Xa oder Thrombin, welches eine photometrisch meßbare austretende Gruppe aufweist,
4) Calciumionen und
5) die weiteren Metallionen;
B) Bestimmung der Umwandlungsrate des exogenen Substrats: und
C) Vergleich der Umwandlungsrate mit der Umwandlungsrate der normalen menschlichen Blutprobe, die in gleicher Weise bestimmt worden ist.

8. Verfahren nach einem der vorhergehenden Ansprüche, dadurch gekennzeichnet, daß die Blutprobe Blut oder eine von Blut abgeleitete Probe, wie eine Blutplasmaprobe oder eine Blutserumprobe, ist.

9. Verfahren nach einem der Ansprüche 4 bis 8, dadurch gekennzeichnet, daß die Stufen 1) bis 5) der Inkubationsstufe A), d. h. 1) die Zugabe eines Aktivators für endogenes Protein C, oder von exogenem aktiviertem Protein C, oder von exogenem Protein C zusammen mit einem Aktivator für Protein C, 2) die Zugabe eines geeigneten Gerinnungsaktivators zur mindestens teilweisen Aktivierung der Gerinnung, 3) Zugabe eines exogenen synthetischen Substrats für entweder Faktor Xa oder Thrombin, 4) Zugabe von Calciumionen bzw. 5) Zugabe der weiteren Metallionen, getrennt und/oder gleichzeitig erfolgen können.

10. Verfahren nach einem der vorhergehenden Ansprüche, gekennzeichnet durch die Zugabe der weiteren Metallionen in der Protein C-Aktivierungsstufe.

11. Verfahren nach einem der vorhergehenden Ansprüche, dadurch gekennzeichnet, daß die Calclumionen in einer Konzentration von 0,5 bis 20 mMol/l, vorzugsweise 1 bis 10 mMol/l des endgültigen Probenmediums verwendet werden.

12. Verfahren nach einem der vorhergehenden Ansprüche, dadurch gekennzeichnet, daß die Mg²⁺-Ionen in einer Konzentration von 20 *µ*Mol/l bis 10 mMol/l, vorzugsweise 100 *µ*Mol/l bis 2 mMol/l und noch bevorzugter 200 *µ*Mol/l bis 1 mMol/l des endgültigen Probenmediums verwendet werden.

13. Verfahren nach einem der vorhergehenden Ansprüche, dadurch gekennzeichnet, daß die Mn²⁺-, Zn²⁺-, Cu²⁺-, Ni²⁺-, Sr²⁺- und/oder Cu⁺-Ionen in einer Konzentration von 1 *µ*Mol/I - 2 mMol/l, vorzugsweise 5 bis 400 *µ*Mol/l und noch bevorzugter 10 bis 80 *µ*Mol/l des endgültigen Probenmediums verwendet werden.

14. Verfahren nach einem der vorhergehenden Ansprüche, dadurch gekennzeichnet, daß die Aktivierung des Proteins C in der Probe der Aktivierung der Gerinnung vorausgeht oder gleichzeitig mit ihr erfolgt.

15. Verfahren nach einem der vorhergehenden Ansprüche, dadurch gekennzeichnet, daß ein Protein C-Aktivator ausgewählt aus der Gruppe, die Protein C aktivierende Schlangengiftenzyme und Thrombin, gewünschtenfalls Thrombin in Kombination mit Thrombomodulin, umfaßt, verwendet wird.

16. Verfahren nach Anspruch 15, dadurch gekennzeichnet, daß ein Protein C aktivierendes Schlangengiftenzym, gewonnen aus der Schlangenfamilie Agkistrodon, vorzugsweise von *Agkistrodon contortrix contortrix* verwendet wird.

17. Verfahren nach Anspruch 16, dadurch gekennzeichnet, daß das rohe Gift oder das Schlangengiftenzympräparat, wie das Schlangengiftenzym ausgewählt aus der Schlangenfamilie Agkistrodon (Protac® C) verwendet wird.

18. Verfahren nach Anspruch 17, gekennzeichnet durch die Verwendung des Protein C-Aktivators, beispielsweise als Schlangengiftenzym ausgewählt von der Schlangenfamilie Agkistrodon (Protac® C), in einer Menge von 1x10⁻³ bis 1 U/ml. vorzugsweise 2x10⁻³ bis 0,3 U/ml in dem endgültigen Probenmedium.

19. Verfahren nach einem der vorhergehenden Ansprüche, gekennzeichnet durch die Verwendung von Zusammensetzungen umfassend
a) Phospholipid(e) und
b) Kontaktaktivatoren und/oder aktivierte Faktoren IX, XII oder XI oder ein Reagens, welches aktivierte Faktoren IX, XII oder XI in vitro bildet, als geeignete Gerinnungsaktivatoren für den intrinsischen Weg.

20. Verfahren nach Anspruch 19, gekennzeichnet durch die Verwendung eines gereinigten Phospholipids oder Mischungen davon oder rohen Extrakten von Tiergeweben. Extrakten von Gehirn, Plazenta, Eidotter oder von Sojabohnen, als Phospholipid(e).

21. Verfahren nach den Ansprüchen 19 und 20, gekennzeichnet durch die Verwendung eines Kontaktaktivators ausgewählt aus der Gruppe, die Ellagsäure. Collagen, Collagen-verwandte Substanzen oder Siliciumdioxid, wie mikronisiertes Siliciumdioxid, colloidales Siliciumdioxid und Kaolin, umfaßt.

22. Verfahren nach einem der vorhergehenden Ansprüche, gekennzeichnet durch die Verwendung von nativem oder rekombinantem menschlichem oder nichtmenschlichem Gewebefaktor (Thromboplastin) von menschlichen oder nichtmenschlichen Spezies mit oder ohne Faktor VII/VIIa oder (nativem oder rekombinantem menschlichem oder nichtmenschlichem) Faktor VIIa und Phospholipiden als geeigneten Gerinnungsaktivator für den extrinsischen Weg.

23. Verfahren nach einem der vorhergehenden Ansprüche, gekennzeichnet durch die Verwendung von exogenem aktiviertem Faktor X oder exogenem Faktor X und exogenem Aktivator für Faktor X oder einem exogenen Aktivator für endogenen Faktor X als geeigneten Gerinnungsaktivator für den gemeinsamen Weg.

24. Verfahren nach Anspruch 23, gekennzeichnet durch die Verwendung eines Schlangengiftenzyms von Russelli viperil als exogenem Aktivator für Faktor X.

25. Verfahren nach einem der vorhergehenden Ansprüche, dadurch gekennzeichnet, daß die Bestandteile des antikoagulierenden Protein C-Wegs dem Reaktionsmedium zugesetzt werden, um variierende funktionelle Gehalte solcher Bestandteile in der Probe zu kompensieren.

26. Verfahren nach Anspruch 25, gekennzeichnet durch die Verwendung von Komponenten des antikoagulatorischen Protein C-Wegs ausgewählt aus der Gruppe, die Protein C, aktiviertes Protein C, Protein S, Faktor V/Faktor Va, oder einem Plasma, das frei von dem zu messenden Bestandteil des tatsächlichen antikoagulatorischen Protein C-Wegs ist, oder eines Plasmas, das frei von sämtlichen Bestandteilen des antikoagulatorischen Protein C-Wegs ist.

27. Verfahren nach einem der vorhergehenden Ansprüche, dadurch gekennzeichnet, daß ein Fibrin-Polymerisationsinhibitor, wie Gly-Pro-Arg-Pro, zu dem Reaktionsmedium zugesetzt wird.

28. Verfahren nach einem der vorhergehenden Ansprüche, dadurch gekennzeichnet, daß Gerinnungsfaktoren ausgewählt aus der Gruppe, die Faktor VIII/ VIIIa, Faktor IX, Faktor X und Prothrombin umfaßt, zu dem Reaktionsmedium zugesetzt werden.

29. Verfahren nach einem der vorhergehenden Ansprüche, dadurch gekennzeichnet, daß die verwendeten Gerinnungsfaktoren ausgewählt werden aus menschlichen oder nichtmenschlichen Quellen oder hergestellt worden sind durch rekombinante Technologie als Wild-Typ-Proteine oder als zur Erlangung der geeigneten funktionellen Wirkung modifizierten Polypeptiden.

30. Verfahren nach einem der vorhergehenden Ansprüche, gekennzeichnet durch die Verwendung eines exogenen synthetischen Substrats für entweder Faktor Xa oder Thrombin in der Reaktionsmischung.

31. Verfahren nach Anspruch 30, dadurch gekennzeichnet, daß als exogenes synthetisches Substrat für entweder Faktor Xa oder Thrombin ein photometrisches Substrat, welches einen Chromophor, Fluorophor oder Luminophor als austretende Gruppe aufweist, verwendet wird.

32. Verfahren nach Anspruch 31, dadurch gekennzeichnet, daß ein photometrisches Substrat, welches eine p-Nftroanilingruppe (pNA) als chromophore austretende Gruppe, eine Naphthylamin-oder Coumarin-Derivatgruppe als fluorophore austretende Gruppe und eine Isoluminolamidgruppe als luminophore austretende Gruppe aufweist, verwendet wird.

33. Verfahren nach Ansprüche 30 bis 32, dadurch gekennzeichnet, daß als Substrat für Faktor Xa Benzoyl-Ile-Glu-Gly-Arg-pNA (S-2222), N-a-Z-D-Arg-Gly-Arg-pNA (S-2765), CH₃SO₂-D-Leu-Gly-Arg-pNA (CBS 31.39) oder MeO-CO-D-CHG-Gly-Arg-pNA (Spektroenzym Xa) verwendet wird.

34. Verfahren nach Anspruch 30 bis 32, dadurch gekennzeichnet, daß als Substrat für Thrombin H-D-Phe-Pip-Arg-pNA (S-2238), pyroGlu-Pro-Arg-pNA (S-2366), H-D-Ala-Pro-Arg-pNA (S-2846), Z-D-Arg-Sarc-Arg-pNA (S-2796), AcOH* H-D-CHG-But-Arg-pNA (CBS 34.47) oder H-D-HHT-Ala-Arg-pNA (Spektroenzym TH) verwendet wird.

35. Kit zur Durchführung des Verfahrens nach einem der vorhergehenden Ansprüche umfassend die folgenden Bestandteile:
a) einen Aktivator für das Protein C; oder exogenes aktiviertes Protein C oder exogenes Protein C zusammen mit einem Aktivator für Protein C;
b) einen geeigneten Gerinnungsaktivator;
c) ein exogenes synthetisches Substrat für entweder Faktor Xa oder Thrombin, welches eine photometrisch meßbare austretende Gruppe aufweist;
d) Calciumionen; und
e) die weiteren Metallionen; in getrennten Behältern und/oder Behältern, welche Mischung von mindestens zwei der Bestandteile in wäßriger Lösung oder in gefriergetrockneter Form enthalten.

36. Kit nach Anspruch 35, dadurch gekennzeichnet, daß er zusätzlich f) Gerinnungsfaktoren umfaßt.

37. Reagens zur Verwendung bei den Verfahren nach einem der Ansprüche 1 bis 34, umfassend mindestens zwei der folgenden Bestandteile a) bis f):
a) einen Aktivator für das Protein C; oder exogenes aktiviertes Protein C oder exogenes Protein C zusammen mit einem Aktivator für Protein C;
b) einen geeigneten Gerinnungsaktivator;
c) ein exogenes synthetisches Substrat für entweder Faktor Xa oder Thrombin, welches eine photometrisch meßbare austretende Gruppe aufweist;
d) Calciumionen;
e) die weiteren Metallionen; und
f) Gerinnungsfaktoren;
dadurch gekennzeichnet, daß es die weiteren Metallionen in Kombination mit einem Protein C-Aktivator, wie Schlangengiftenzym ausgewählt aus der Schlangenfamilie Agkistrodon (Protac® C) oder Thrombin/Thrombomodulin, in einem Behälter in wäßriger Lösung oder in gefriergetrockneter Form umfaßt.

## Revendications

1. Procédé photométrique in vitro de dépistage qualitatif et de détermination quantitative de l'activité fonctionnelle des composants de la voie de coagulation sanguine à anticoagulant de type protéine C, comprenant une mesure du taux de conversion d'un substrat exogène par une enzyme dont l'activité est liée à l'activité anticoagulante de la protéine C, dans un échantillon de sang humain comprenant des facteurs de coagulation et ledit substrat exogène après une activation au moins partielle de la coagulation selon la voie intrinsèque, extrinsèque ou commune, et un déclenchement de coagulation par addition d'ions calcium ; et une comparaison dudit taux de conversion avec le taux de conversion d'un échantillon de sang humain déterminé de la même façon, caractérisé en ce qu'on ajoute d'autres ions d'un ou plusieurs métaux dans ledit échantillon.

2. Procédé selon la revendication 1,
caractérisé en ce que l'on utilise des ions de métal divalents ou des ions cuivre monovalents en tant que lesdits autres ions d'un ou plusieurs métaux.

3. Procédé selon la revendication 1 ou 2,
caractérisé en ce que l'on emploie des ions Mg²⁺, Mn²⁺, Zn²⁺, Cu²⁺, Ni²⁺, Sr²⁺ et/ou Cu⁺ en tant que lesdits autres ions d'un ou plusieurs métaux.

4. Procédé selon les revendications 1 à 3 pour le dépistage global des défauts dans la voie de coagulation sanguine à anticoagulation de type protéine C chez un homme, selon lequel :
A) on incube un échantillon de sang dudit homme comprenant des facteurs de coagulation avec
1) un activateur de la protéine C dans ledit échantillon,
2) un activateur de coagulation approprié,
3) un substrat synthétique exogène pour le facteur Xa ou la thrombine, comprenant un groupe partant dosable par voie photométrique,
4) des ions calcium, et
5) lesdits autres ions d'un ou plusieurs métaux ;
B) on détermine le taux de conversion dudit substrat exogène ; et
C) on compare ledit taux de conversion avec ledit taux de conversion d'un échantillon de sang humain normal déterminé de la même façon.

5. Procédé selon les revendications 1 à 3 pour la détermination de l'activité de la protéine S libre dans un échantillon de sang humain, selon lequel :
A) on incube ledit échantillon de sang comprenant des facteurs de coagulation avec :
1) de la protéine C exogène activée ou de la protéine C exogène en association avec un activateur de protéine C,
2) un activateur de coagulation approprié,
3) un substrat synthétique exogène pour le facteur Xa ou la thrombine, comprenant un groupe partant dosable par voie photométrique,
4) des ions calcium, et
5) lesdits autres ions d'un ou plusieurs métaux ;
B) on détermine le taux de conversion dudit substrat exogène ; et
C) on compare ledit taux de conversion avec le taux de conversion d'un échantillon de sang humain normal déterminé de la même façon.

6. Procédé selon les revendications 1 à 3 pour la détermination de l'activité de la protéine C dans un échantillon, de sang dudit homme, selon lequel :
A) on incube un échantillon de sang humain comprenant des facteurs de coagulation avec :
1) un activateur pour la protéine C dans ledit échantillon,
2) un activateur de coagulation approprié,
3) un substrat synthétique exogène pour le facteur Xa ou la thrombine, comprenant un groupe partant dosable par voie photométrique,
4) des ions calcium, et
5) lesdits autres ions d'un ou plusieurs métaux ;
B) on détermine le taux de conversion dudit substrat exogène ; et
C) on compare ledit taux de conversion avec le taux de conversion d'un échantillon de sang humain normal déterminé de la même façon.

7. Procédé selon les revendications 1 à 3 pour dépister une ou les mutations du facteur V dans un échantillon de sang humain, selon lequel :
A) on incube un échantillon de sang dudit homme comprenant des facteurs de coagulation avec :
1) de la protéine C exogène activée, ou de la protéine C exogène associée à un activateur de protéine C ou un activateur de protéine C endogène,
2) un activateur de coagulation approprié,
3) un substrat exogène synthétique pour le facteur Xa ou la thrombine, comprenant un groupe partant dosable par voie photométrique,
4) des ions calcium, et
5) lesdits autres ions d'un ou plusieurs métaux ;
B) on détermine le taux de conversion dudit substrat exogène ; et
C) on compare ledit taux de conversion avec le taux de conversion d'un échantillon de sang humain normal déterminé de la même façon.

8. Procédé selon l'une quelconque des revendications précédentes, caractérisé en ce que l'échantillon de sang est du sang ou un échantillon dérivé du sang tel qu'un échantillon de plasma sanguin ou un échantillon de sérum sanguin.

9. Procédé selon l'une quelconque des revendications 4 à 8, caractérisé en ce que lesdites phases 1 à 5 de l'étape d'incubation A), c'est-à-dire 1) l'addition d'un activataur pour la protéine C endogène, ou de protéine C exogène activée, ou de protéine C exogène ensemble avec un activateur de protéine C, l'addition d'un activateur de coagulation approprié pour obtenir une activation au moins partielle de la coagulation, 3) l'addition d'un substrat synthétique exogène pour le facteur Xa ou la thrombine, 4) l'addition d'ions calcium, et 5) l'addition desdits autres ions d'un ou plusieurs métaux, peuvent être respectivement effectuées séparément et/ou simultanément.

10. Procédé selon l'une quelconque des revendications précédentes, caractérisé en ce qu'on ajoute lesdits autres ions d'un ou plusieurs métaux dans la phase d'activation de la protéine C.

11. Procédé selon l'une quelconque des revendications précédentes, caractérisé en ce que lesdits ions calcium sont employés selon une concentration de 0,5 à 20 mmoles/l, de préférence de 1 à 10 mmoles/l du milieu d'essai final.

12. , Procédé selon l'une quelconque des revendications précédentes, caractérisé en ce que lesdits ions Mg²⁺ sont employés selon une concentration de 20 µmoles/l à 10 mmoles/l, de préférence de 100 µmoles/l à 2 mmoles/l et en particulier de 200 µmoles/l à 1 mmole/l du milieu d'essai final.

13. Procédé selon l'une quelconque des revendications précédentes, caractérisé en ce que lesdits ions Mn²⁺, Zn³⁺, Cu²⁺, Ni²⁺, Sr²⁺ et/ou Cu⁺ sont employés selon une concentration de 1 µmole/l à 2 mmoles/1, de préférence de 5 à 400 µmoles/l, et en particulier de 10 à 80 µmoles/l du milieu d'essai final.

14. Procédé selon l'une quelconque des revendications précédentes, caractérisé en ce que l'activation de la protéine C dans ledit échantillon précède ou se produit en même temps qu'une activation de coagulation.

15. Procédé selon l'une quelconque des revendications précédentes, caractérisé en ce que l'on emploie un activateur de protéine C choisi parmi les enzymes de venin de serpent activant la protéine C et la thrombine, et si on le souhaite de la thrombine associée à de la thrombomoduline.

16. Procédé selon la revendication 15,
caractérisé en ce que l'on utilise une enzyme de venin de serpent activant la protéine C dérivée des serpents de la famille *Agkistrodon*, de préférence de *Agkistrodon contortrix contortrix*.

17. Procédé selon la revendication 16,
caractérisé en ce que l'on emploie le venin brut ou une composition à base d'enzyme de venin de serpent, telle qu'une enzyme de venin de serpent choisie parmi ceux des serpents de la famille *Agkistrodon* (Protac® C).

18. Procédé selon la revendication 17,
caractérisé en ce qu'on utilise l'activateur de protéine C tel qu'une enzyme de venin de serpent choisie parmi celles des serpents de la famille *Agkistrodon* (Protac® C) selon une quantité de 1x10⁻³ à 1 U/ml, de préférence de 2x10⁻³ à 0,3 U/ml dans le milieu d'essai final.

19. Procédé selon l'une quelconque des revendications précédentes, caractérisé en ce que l'on utilise comme activateurs de coagulation appropriés pour la voie intrinsèque, des compositions comprenant :
a) un ou plusieurs phospholipides, et,
b) des activateurs de contact et/ou le facteur IX, XII ou XI activé ou un réactif générant in vitro le facteur IX, XII ou XI activé.

20. Procédé selon la revendication 19,
caractérisé en ce que l'on utilise en tant que phospholipide(s), un phospholipide purifié ou un mélange de ceux-ci, ou des extraits bruts de tissus animaux, des extraits de cerveau, de placenta, de jaune d'oeuf ou de soja.

21. Procédé selon les revendications 19 et 20,
caractérisé en ce que l'on emploie l'activateur de contact choisi parmi l'acide ellagique, le collagène, des substances apparentées au collagène ou une silice telle que de la silice micronisée, de la silice colloïdale et le kaolin.

22. Procédé selon l'une quelconque des revendications précédentes, caractérisé en ce que l'on utilise un facteur tissulaire (thromboplastine) natif ou recombinant, humain ou non humain, dérivé d'une espèce humaine ou non humaine, avec ou sans facteur VII ou VIIa, ou du facteur VIIa (natif ou recombinant et humain ou non humain) et des phospholipides en tant qu'activateur de coagulation approprié pour la voie extrinsèque.

23. Procédé selon l'une quelconque des revendications précédentes, caractérisé en ce que l'on emploie du facteur X exogène activé, ou du facteur X exogène et un activateur exogène pour le facteur X, ou un activateur exogène pour le facteur X endogène en tant qu'activateur de coagulation approprié pour la voie commune.

24. Procédé selon la revendication 23,
caractérisé en ce que l'on utilise une enzyme de venin de serpent dérivée de *Viperii Russelli* en tant qu'activateur exogène du facteur X.

25. Procédé selon l'une quelconque des revendications précédentes, caractérisé en ce que l'on ajoute des composants de la voie à anticoagulant de type protéine C, dans le milieu réactionnel afin de compenser les niveaux fonctionnels variables de ces composants dans l'échantillon.

26. Procédé selon la revendication 25,
caractérisé en ce que l'on utilise des composants de la voie à anticoagulant de type protéine C choisis parmi la protéine C, la protéine C activée, la protéine S, le facteur V/facteur Va, ou un plasma déficient en le composant considéré de la voie à anticoagulant de type protéine C destiné à être dosé, ou un plasma déficient en tous lesdits composants de la voie à anticoagulant de type protéine C.

27. Procédé selon l'une quelconque des revendications précédentes, caractérisé en ce que l'on ajoute un inhibiteur de polymérisation de fibrine tel que Gly-Pro-Arg-Pro dans le milieu réactionnel.

28. Procédé selon l'une quelconque des revendications précédentes, caractérisé en ce que l'on ajoute des facteurs de coagulation choisis parmi le facteur VIII/facteur VIIIa, le facteur IX, le facteur X et la prothrombine dans le milieu réactionnel.

29. Procédé selon l'une quelconque des revendications précédentes, caractérisé en ce que les facteurs de coagulation employés sont d'origine humaines et non humaines, ou sont produits par recombinaison sous forme de protéines de type sauvage ou de polypeptides modifiés, pour obtenir la propriété fonctionnelle requise.

30. Procédé selon l'une quelconque des revendications précédentes, caractérisé en ce que l'on emploie un substrat synthétique exogène pour le facteur Xa ou la thrombine dans le mélange réactionnel.

31. Procédé selon la revendication 30,
caractérisé en ce que comme substrat synthétique exogène pour le facteur Xa ou la thrombine, on utilise un substrat photométrique comprenant un chromophore, un fluorophore ou un luminophore en tant que groupe partant.

32. Procédé selon la revendication 31,
caractérisé en ce que l'on emploie un substrat photométrique comprenant un groupe p-nitroaniline (pNa) comme groupe partant chromophore, un groupe dérivé de naphtylamine ou de coumarine en tant que groupe partant fluorophore, et un groupe isoluminolamide en tant que groupe partant luminophore.

33. Procédé selon les revendications 30 à 32,
caractérisé en ce que l'on emploie comme substrat pour le facteur Xa : Benzoyl-Ile-Glu-Gly-Arg-pNA (S-2222), N-a-Z-D-Arg-Gly-Arg-pNa (S-2765), CH₃SO₂-D-Leu-Gly-Arg-pNa (CES 31.39) ou MeO-CO-D-CHG-Gly-Arg-pNa.

34. Procédé selon les revendications 30 à 32,
caractérisé en ce que l'on emploie comme substrat pour la thrombine ; H-D-Phe-Pip-Arg-pNA (S-2238), pyroGlu-Pro-Arg-pNA (S-2366), H-D-Ala-Pro-Arg-pNA (S-2846), Z-D-Arg-Sarc-Arg-pNA (S-2796), AcOH^{*}H-D-CHG-But-Arg-pNA (CBS 34.37). ou H-D-HHT-Ala-Arg-pNA (Spectrozyme TH).

35. Nécessaire destiné à être utilisé dans les procédés de l'une quelconque des revendications précédentes, comprenant les composants suivants :
a) un activateur pour la protéine C ; ou de la protéine C exogène activée, ou de la protéine C exogène associée avec un activateur de protéine C ;
b) un activateur de coagulation approprié ;
c) un substrat synthétique exogène pour le facteur Xa ou la thrombine, comprenant un groupe partant dosable par voie photométrique ;
d) des ions calcium ; et
e) lesdits autres ions d'un ou plusieurs métaux ;
dans des récipients séparés et/ou dans des récipients comprenant des mélanges d'au moins deux desdits composants en solution aqueuse ou sous forme lyophilisée.

36. Nécessaire selon la revendication 35,
caractérisé en ce qu'il comprend en outre f) des facteurs de coagulation.

37. Réactif destiné à être utilisé dans les procédés de l'une quelconque des revendications 1 à 34, comprenant au moins deux des composants a) à f) suivants :
a) un activateur pour la protéine C ; ou de la protéine C exogène activée, ou de la protéine C exogène associée avec un activateur de protéine C ;
b) un activateur de coagulation approprié ;
c) un substrat synthétique exogène pour le facteur Xa ou la thrombine, comprenant un groupe partant dosable par voie photométrique ;
d) des ions calcium ;
e) lesdits autres ions d'un ou plusieurs métaux ; et
f) des facteurs de coagulation ;
caractérisé en ce qu'il comprend lesdits autres ions d'un ou plusieurs métaux en association avec un activateur de protéine C, tel qu'une enzyme de venin de serpent choisie parmi celles des serpents de la famille Agkistrodon (Protac® C) ou la thrombine/thrombomoduline, dans un récipient en solution aqueuse ou sous forme lyophilisée.
